# EUROPEAN PATENT SPECIFICATION

(11) **EP 2 499 117 B1**
(45) Date of publication and mention of the grant of the patent: **21.10.2015**
(21) Application number: 10781867.6
(22) Date of filing: 12.11.2010
(51) Int. Cl.: C07D 235/14, C07D 403/12, A61K 31/4184, A61P 35/00, A61K 31/4178, A61K 31/4196, A61K 31/496, A61K 31/506, A61K 31/5377, A61K 31/55, A61K 31/551, C07D 235/30, C07D 417/12, C07D 471/04, C07D 401/12

(54) **A COMPOUND, A PROCESS FOR ITS PREPARATION, A PHARMACEUTICAL COMPOSITION, USE OF A COMPOUND, A METHOD FOR MODULATING OR REGULATING SERINE/THREONINE KINASES AND A SERINE/THREONINE KINASES MODULATING AGENT**
Verbindung, Verfahren zu deren Herstellung, pharmazeutische Zusammensetzung, Verwendung einer Verbindung, Verfahren zur Modulierung und Regulierung von Serin-/Threoninkinasen sowie ein Modulierungsmittel für Serin-/Threoninkinasen
Composé, son procédé de préparation, composition pharmaceutique, utilisation d'un composé, procédé de modulation ou de régulation de serine/thréonine kinases et agent de modulation de serine/thréonine kinases

(30) Priority: 12.11.2009 EP 09460048; 12.11.2009 US 617067
(43) Date of publication of application: 19.09.2012
(73) Proprietor: SELVITA S.A., 30 348 Krakow (PL)
(72) Inventor: BRZÓZKA, Krzysztof, 30-498 Kraków (PL); CZARDYBON, Wojciech, 43 190 Mikolow (PL); SABINIARZ, Aleksandra, 56 120 Brzeg Dolny (PL); MILLIK, Mariusz, 30-396 Kraków (PL); WINDAK, Renata, 30 544 Kraków (PL); ZAREBSKI, Adrian, 30-383 Kraków (PL); BEUZEN, Nicolas, 30 348 Kraków (PL)
(74) Representative: Bühler, Dirk
(86) International application number: PCT/EP2010/067385
(87) International publication number: WO 2011/058139

(56) References cited:
- WO-A1-02/46169
- WO-A1-2005/004864
- WO-A1-2007/062342
- WO-A1-2007/100646
- WO-A1-2008/003857
- GB-A- 1 286 603
- US-A- 3 705 944
- US-A- 3 935 314
- US-A1- 2004 006 119
- US-A1- 2009 197 889
- RÖCHLING H ET AL: "Hemmstoffe der Photosynthese VII. Synthesen von Benzimidazolonen und polychlorierten Benzimidazolen", ZEITSCHRIFT FUR NATURFORSCHUNG, TEIL B:ANORGANISCHE CHEMIE, ORGANISCHE CHEMIE, VERLAG DER ZEITSCHRIFT FUR NATURFORSCHUNG. TUBINGEN, DE, vol. 25, 1 January 1970 (1970-01-01), pages 954-960, XP009144201, ISSN: 0932-0776
- WERBEL L M ET AL: "SYNTHESIS AND ANTIMALARIAL EFFECTS OF 5,6-DICHLORO-2-Ä(4-ÄÄ4-(DIETHYL AMINO)-1-METHYLBUTYLÜAMINOÜ-6-METHYL-2-PYR IMIDINYL)AMINOÜBENZIMIDAZOLE AND RELATED BENZIMIDAZOLES AND 1H-IMIDAZOÄ4,5-BÜPYRIMIDINE (1,2)", 1 June 1973 (1973-06-01), JOURNAL OF HETEROCYCLIC CHEMISTRY, WILEY-BLACKWELL PUBLISHING, INC, US, PAGE(S) 363 - 382, XP009001685, ISSN: 0022-152X examples 4, 11, 54; tables I, II, III
- KOLESNIKOVA I V ET AL: "REACTION OF N-PENTAFLUOROPHENYLCARBONIMIDOYL DICHLORIDE WITH PRIMARY AMINES", JOURNAL OF ORGANIC CHEMISTRY OF THE USSR, M A I K NAUKA - INTERPERIODICA, RU, vol. 25, no. 8, 1 August 1989 (1989-08-01) , pages 1523-1529, XP002914637, ISSN: 0022-3271
- KOLESNIKOVA T V ET AL: "REACTIONS OF N-POLYFLUOROPHENYLCARBONIMIDOYL DICHLORIDES WITH PRIMARY AND SECONDARY AMINES, KINETICS AND MECHANISM. SYNTHESIS OF POLYFLUORINATED CARBODIIMIDES, CHLOROFORMAMIDINES, GUANIDINES AND BENZIMIDAZOLES", JOURNAL OF FLUORINE CHEMISTRY, ELSEVIER, NL, vol. 40, 1 January 1988 (1988-01-01), pages 217-246, XP002914639, ISSN: 0022-1139, DOI: DOI:10.1016/S0022-1139(00)83067-5
- MURATA, H.; MIYAZAKI, Y.; INABA, A.; PADUAN-FILHO, A.; BINDILATTI, V.; FERNANDES OLIVEIRA JR., N.; DELEN, Z.; LAHTI, P.M.: "2-(4,5,6,7-Tetrafluorobenzimidazol-2-yl)- 4,4,5,5-tetramethyl-4,5-dihydro-1-H-imidaz ole-3-oxide-1-oxyl, A Hydrogen-Bonded Organic Quasi-1D Ferromagnet", JOURNAL OF THE AMERICAN CHEMICAL SOCIETY, vol. 130, 2008, pages 186-194, XP002621129, ISSN: 0002-7863, DOI: 10.1021/ja074211g
- INUKAI, Y.; OONO, Y.; SONODA, T.; KOBAYASHI, H.: "ortho-Disubstituted F-Benzenes. I. Preparation of (F-Benzo)heterocyclic Compounds from F-Aniline and the Reactions of Some Intermediate (F-Phenyl)amino Compounds", BULLETIN OF THE CHEMICAL SOCIETY OF JAPAN, vol. 52, no. 2, 1979, pages 516-520, XP002621130, ISSN: 1348-0634
- DATABASE REGISTRY [Online] CHEMICAL ABSTRACTS SERVICE, COLUMBUS, OHIO, US; 3 December 2004 (2004-12-03), "1H-Benzimidazol-2-amine, 4,5 6,7-tetrafluoro-N-(4-methyl-3-thienyl)-", XP002621131, Database accession no. 791780-63-7
- DATABASE REGISTRY [Online] CHEMICAL ABSTRACTS SERVICE, COLUMBUS, OHIO, US; 2 December 2004 (2004-12-02), "1H-Benzimidazol-2-amine, N-(2-chloro-4-methyl-3-thienyl)-4,5,6,7-te trafluoro-", XP002621132, Database accession no. 791584-58-2
- COATS E A ET AL: "CORRELATION ANALYSIS OF PYRIMIDINE FOLIC ACID ANTAGONISTS AS ANTIBACTERIAL AGENTS .I", EUROPEAN JOURNAL OF MEDICINAL CHEMISTRY, EDITIONS SCIENTIFIQUE ELSEVIER, PARIS, FR, vol. 14, no. 3, 1 May 1979 (1979-05-01), pages 261-270, XP000985014, ISSN: 0223-5234
- GIANONCELLI, A.; COZZA, G.; ORZESZKO, A.; MEGGIO, F.; KAZIMIERCZUK, Z.; PINNA, L.A.: "Tetraiodobenzimidazoles are potent inhibitors of protein kinase CK2", BIOORGANIC & MEDICINAL CHEMISTRY, vol. 17, 27 August 2009 (2009-08-27), pages 7281-7289, XP002621133, cited in the application
- IUPAC ED - ALAN D MCNAUGHT AND ANDREW WILKINSON: "alkyl groups", [Online] 1 January 1997 (1997-01-01), COMPENDIUM OF CHEMICAL TERMINOLOGY : IUPAC RECOMMENDATIONS; [IUPAC CHEMICAL DATA SERIES], BLACKWELL SCIENCE, OXFORD [U.A.], XP002585005, ISBN: 978-0-86542-684-9 Retrieved from the Internet: URL:http://www.iupac.org/goldbook/A00228.p df> [retrieved on 1997-01-01] the whole document

## Description

### FIELD OF THE INVENTION

The present invention is inter alia concerned with a compound, a process for its preparation, a pharmaceutical composition, use of a compound, a method for modulating or regulating serine/threonine and tyrosine kinases and a serine/threonine and tyrosine kinases modulating agent. The present invention thus inter alia relates to novel small-molecule compounds with kinase inhibitory activity, having superior properties as pharmaceutical agents, production method thereof and uses thereof. In particular, this invention relates to new derivatives of tetrahalogenated benzimidazoles with serine/threonine and tyrosine kinases inhibitory properties, wherein the kinases are preferably selected from the group of PIM, HIPK, DYRK, CLK, CDK, FLT, PKG, Haspin, MER, TAO, MNK, TRK kinases, and wherein the derivatives exhibit inter alia superior pharmacological actions, and can be useful for the treatment of disease conditions, especially cancers depending on serine/threonine kinases, such as e.g. leukemias, lymphomas and solid tumors.

### BACKGROUND OF THE INVENTION

Kinases are enzymes that modify other proteins by chemically adding phosphate groups to them (a process called phosphorylation). Phosphorylation of the targeted proteins results in a functional change of their activity but also can modify association with other proteins, traffiking and subcellular localization. It is estimated that up to 30% of all proteins can be modified by kinases. For this reason kinases are key regulators of majority of cellular pathways, especially those involved in signal transduction. Kinases are currently one of the most interesting and most extensively investigated drug targets. Among the new kinase targets for therapeutic inhibition pursued currently, PIM kinases are definitely one of the most interesting emerging molecular targets. The PIM family of serine-threonine kinases is composed of three highly homologous proteins PIM-1, -2 and -3 which play an important role in intracellular signaling and contribute to pathways involved in cell survival, inflammation, cell movement and stress response.

Around 15 years ago, the *PIM-1* gene was identified as a proviral insertion site of the Moloney Murine Leukemia Virus (MoMuLV) in experiments designed to find new genes, which are involved in tumorogenesis. These findings established *PIM-1* as a proto-oncogene and important player in the process of malignant transformation (Nagarajan *et al*., 1986). In humans, the *PIM-1* gene is located on chromosome 6p21.1-p21.31 (Zakut-Houri *et al.,* 1987). The *PIM-1* gene comprises approximately 5 kb, contains six exons and five introns. Its cDNA contains an open reading frame of 313 codons with 94% homology to the mouse counterpart. The RNA transcript is 2.9 kilobases (kb) long (Saris *et al*., 1991). Shortly after discovery of the gene, PIM-1 protein was identified to be a serine/ threonine kinase, belonging to CAMK kinases (group of calcium/calmodulin-regulated kinases). (Saris *et al*., 1991; Reeves *et al*., 1990). It contains ATP-anchor, kinase domain and an active site. There are two forms of PIM-1 in human and mouse, associated with alternative initiation codon (44kDa and 33kDa). Both forms have relatively short half-life, however the 44kDa isoform seems to be more stable. Activity of PIM-1 kinase is found in the cytoplasm, nuclear fraction and the membrane of the cells. Two isoforms have different subcellular localization - 44kDa is present mainly on the plasma membrane; 33kDa in nucleus and cytoplasm. The crystal structure of PIM-1 obtained in 2005 revealed that it is a constitutively active kinase and that in contrast to many other kinases, like Akt or MAPK kinases, PIM-1 does not require additional phosphorylation for its kinase activity, however phosphorylation of this protein may contribute to its stability (Qian *et al*., 2005a; Qian *et al*., 2005b). Obtained crystal structure allowed also discovering unusual structural features of the PIM-1 kinase domain. Most notably, the hinge region presents two features of particular interest: an insertion residue as well as a proline residue (Pro123), which combine to form an ATP- and inhibitor-binding region quite distinct from other protein kinases. A substrate recognition sequence of the PIM-1 kinase was identified by selective peptide mapping ((K/R)3-X-S/T-X or R/K-R/K-R-R/K-X-S/T-X, where X is an amino acid (aa) residue with a small side chain but neither basic nor acidic) (Peng *et al*., 2007). With regard to molecular mechanisms of PIM-1 involvement in oncogenic transformation and cancer development, one can point out several processes that are regulated by the PIM-1 kinase like stimulation of cell cycle progression, coactivation of mTOR pathway, inhibition of apoptosis, transcriptional coactivation of c-Myc, promotion of drug resistance and cell migration and metastasis PIM kinases overexpression has been reported in a variety of cancer types, ranging from hematopoietic malignancies such as diffuse B cell lymphoma, chronic lymphocytic leukemia and acute myelogenous leukemia to solid tumors such as prostate and pancreatic cancer. Acquisition of mutations in the *PIM-1* gene can be one of the molecular mechanisms involved in histological transformation of follicular lymphoma (FL) and B-chronic lymphocytic leukemia (B-CLL) to diffuse large B-cell lymphoma (DLBCL)(Rossi *et al.,* 2006). Mutations of the *PIM-1* gene have also been detected in cases of AIDS-associated non-Hodgkin lymphoma (Gaidano *et al.,* 2003), HCV-infected B-cell NHL patients (Libra *et al*., 2005), primary central nervous system lymphomas (PCNSLs) (Montesinos-Rongen *et al*., 2004), extranodal DLBCL cases and primary cutaneous marginal zone B-cell lymphoma (PCMZL) (Deutsch *et al*., 2009; Deutsch *et al*., 2007), primary mediastinal large B-cell lymphoma (PMLBCL)(Martelli *et al*., 2008). PIM-1 kinase is upregulated in Epstein Barr virus infected B-cells where it enhances transcriptional activity of EBNA2 protein, essential for the growth transformation and immortalization of infected B-cells. This mechanism of action of PIM-1 kinases may predispose immortalized B-cell to undergo malignant transformation (Rainio *et al.,* 2005). Initial study performed by Amson in 1989 showed that the 33kDa isoform of PIM-1 kinase was overexpressed in approximately 30% of the analyzed samples (out of 70 hematopoietic malignancies analyzed - 51 patients and 19 cell lines), pointing toward the role of this kinase in development of myeloid and lymphoid acute leukemias (Amson *et al.,* 1989). This finding was further confirmed in a variety of other studies showing elevated levels of PIM-1 kinase in various human clinical leukemias and myeloid and lymphocytic cell lines (Meeker *et al.,* 1990). For example, PIM-1 kinase was implicated in leukemogenesis and aberrant expression levels of this kinase can be involved neoplastic transformation by phosphorylation and activation of Runx transcription factors (Aho *et al.,* 2006; Kim *et al.,* 2008). Chromosome translocations and point mutations are well-documented and frequent genetic alterations in RUNX leukemias (Penther *et al.,* 2002; Osato and Ito, 2005). PIM-1 seems to play also a crucial role in development of acute myeloid leukemias (AML). Several reports pointed out a role of PIM-1 kinase in downstream signaling by FLT3 (Fms-like tyrosine kinase 3) kinase. Constitutively activating internal tandem duplication (ITD) mutations of the receptor tyrosine kinase FLT3 play an important role in leukemogenesis, and their presence is associated with poor prognosis in AML. Constitutive FLT3 signaling upregulates PIM-1 levels in leukemia cells and the juxtamembrane domain of FLT3 is a critical domain required for this upregulation (Kim *et al.,* 2005; Vu *et al.,* 2009). Interestingly, this downstream signaling seems to be independent of STAT5, Akt and MAPK signaling. Up-regulation of PIM-1 kinase contributes to the proliferative and antiapoptotic pathways induced by FLT3 signaling, and the major antiapoptotic mechanism of action is PIM-1 dependent Bad phosphorylation (Kim *et al*., 2006). Similarly to FLT3, PIM-1 kinase is also upregulated by the Bcr-Abl fusion protein, a major cause of the chronic myelogenous leukemia. A SH3 /SH2 mediated interaction of Bcr/Abl kinase with Hck kinase (hematopoietic cell kinase) lead to activation of Hck and phosphorylation of STAT5B on the critical Tyr699 residue. Activated STAT5B stimulates expression of downstream effectors like PIM-1 kinase and the A1 protein, key factors essential for in vitro transformation and in vivo leukemogenesis mediated by Bcr/Abl. (Klejman *et al.,* 2002; Nieborowska-Skorska *et al.,* 2002). Whereas inhibition of PIM-1 seems not to be sufficient to overcome Bcr/Abl mediated transformation in cancer cells, an elegant study by Adam *et al.,* showed that PIM-1 and PIM-2 play here redundant roles and simultaneous targeting of the two kinases may be an exciting therapeutic alternative to overcome resistance against small-molecule tyrosine kinase inhibitors (Nosaka and Kitamura, 2002; Adam *et al.,* 2006). Involvement of PIM-1 kinase in development of prostate cancer has been extensively studied over the past years and provided several examples of clinical importance and rationale for therapeutic indication. Already in 2001 in a microarrays screen PIM-1 expression was shown to correlate with clinical outcome of the disease and was suggested to be a better marker than the standard diagnostic test for PSA levels in serum (Dhanasekaran *et al*., 2001). This was further confirmed in studies performed by other groups (Cibull *et al*., 2006; Xu *et al.,* 2005; Thompson *et al.,* 2003; Valdman *et al.,* 2004). Overexpression of PIM-1 in human prostate cancer cells induces genomic instability by subverting the mitotic spindle checkpoint, centrosome amplification, chromosome misaggregation and polyploidy. When the PIM-1kinase is overexpressed in immortalized, non-tumorigenic human cells, these cells became tumorigenic (Roh *et al*., 2008; Roh *et al.,* 2003). A very interesting finding by Zemskova and colleagues support additionally use of PIM-1 kinase inhibitors in prostate cancer treatment. Surprisingly, treatment of prostate cancer cells with docetaxel, a standard of care induces STAT3 phosphorylation and transcriptional upregulation of the *PIM-1* gene. Expression of PIM-1 kinase was crucial for survival of these cells after docetaxel treatment, as shown by knock down and inhibitor experiments. This data supports further testing of novel, small molecule kinase inhibitors in combination therapies with patients with docetaxel resistance (Zemskova *et al.,* 2008). In an extensive study by Beier *et al.,* immunohistochemistry experiment performed on cells compared to non-neoplastic tissue showed overexpression of the PIM-1 protein in 98% (41/42) of invasive head and neck squamous cell carcinomas (HNSCC). This study was repeated using primary tumors and metastasis biopsies showing nearly significant correlation of PIM-1 expression with histological tumor, underlining role of PIM-1 in HNSCC developments (Beier *et al.,* 2007). In line with this finding, moderate or high expression of PIM-1 and nuclear localization was also linked to prediction of radiation response in squamocellular cancers of head and neck (Peltola *et al.,* 2009).

PIM-2 is a second member of the PIM kinase family. Functionally, it has been noticed that PIM-2 overlaps with the Akt/mTOR pathway, but is regulated independently. Both PIM-2 and Akt1 kinase regulate NFκB-dependent transcription by phosphorylation of the Cot kinase (Kane *et al.,* 2002; Hammerman *et al.,* 2004). It has been indicated that PIM-2 expression maintains high levels of NF-κB activity and NF-κB activation by PIM-2 is required for its antiapoptotic function. Moreover, the data has suggested that Cot-dependent activation of NFκB can occur via the transcriptional induction of PIM-2 rather than as a direct result of a receptor-initiated kinase cascade. Several reports showed that PIM-2 can to some extent substitute or cooperate with PIM-1 in driving tumorigenesis. As both kinases share some of the targets, like the Bad protein, they act both as prosurvival kinases preventing induction of apoptosis (Yan *et al.,* 2003; Aho *et al.,* 2004). As both PIM-1 and 2 are transcriptionally induced by upstream signaling (like FLT3 or Bcr-Abl signaling), they can cooperate and are essential in neoplastic transformation of B-cells by v-Abl oncogene (Chen *et al.,* 2008). Similarly to PIM-1, coexpression of PIM-2 and c-Myc transgene induces malignant transformation (Allen *et al.,* 1997). Also the effect on the cell cycle inhibition for both PIM-1 and PIM-2 seem to synergize in accelerating cell proliferation and cell cycle progression as shown in the literature, although the molecular mechanism of cell cycle regulation are described in detail only for PIM-1 kinase (Dai *et al.,* 2005; Chen *et al.,* 2005) There seem however also to be differences between the two kinases. Whereas recent publications on hypoxia point out its emerging role in solid tumor formation and chemoresistance, no similar reports are known for PIM-2 kinase and this role needs to be explored. On the other hand, in the recent publication by Tamburini, a special emphasis was put on the role of PIM-2 in phosphorylation of crucial 4EBP1 transcription factor (on serine S65)(Tamburini *et al.,* 2009). As shown in this publication, expression of PIM-1 in clinical samples did not correlate with the above finding, providing a proof for nonoverlapping role of PIM-1 and PIM-2 in regulation of 4EBP1 phosphorylation, regulation of protein synthesis and promotion of neoplastic transformation. Similar finding were already reported in by Fox and colleagues, stressing out a crucial role of PIM-2 kinase in controlling translation independently from the Akt/mTOR pathway and pointing towards inhibition of PIM-1 kinase as an attractive option for development of new therapies, especially in acute myelogenous leukemia (Fox *et al.,* 2003).

Similarly to PIM-1, overexpression of PIM-2 has been documented in several human tumors types. One of the distinguishing reports is involvement of PIM-2 in tumorigenesis of hepatocellular carcinoma (HCC) (Gong *et al.,* 2008). PIM-2 gene expression and its protein levels were investigated in human liver cancer tissues and HepG2 cells (human hepatocellular liver carcinoma cell line). In both cases the expression of PIM-2 gene and protein was higher than in immortalized liver cell line L02, indicating its role as a tumor biomarker. Further experiments indicated that PIM-2 expression and its kinase activity are IL-3 dependent; however its apoptotic inhibition role is IL-3-inedependent. It was also found that protection against apoptosis by PIM-2 is glucose-dependent, so liver cells growing *in vivo*, surrounded by high glucose and growth factors concentration have favorable conditions to express PIM-2, however PIM-2 was unable to prevent apoptosis upon glucose deprivation. So once overexpressed in hepatic cells PIM-2 can be an important factor in tumorigenesis.

PIM-3 (also known as Kid-1 - kinase induced by depolarization) is the third member of the PIM kinase family. Similarly to PIM-2 and PIM-1, PIM-3 acts in a prosurvival way preventing apoptosis by phosphorylation of Bad. However, in contrast to PIM-1/2, PIM-3 seems to be less specific to Ser112 residue, preferably phosphorylating Ser136, Ser155 and Ser170 (Macdonald *et al.,* 2006). PIM-3 was the most effective kinase in phosphorylating Ser136 residue, which seems to be crucial for subsequent phosphorylation steps and interaction with the anti-apoptotic Bcl-XL protein. PIM phosphorylation of Bad was therefore found to promote the 14-3-3 binding and inhibition of Bcl-XL binding. Similarly to PIM-1, PIM-3 seems to be also involved in promoting vessel formation and angiogenesis (Zippo *et al.,* 2004; Zhang *et al.,* 2009b). Angiogenesis is a physiological process involving the growth of new blood vessels from pre-existing vessels. This feature play significant role in tumorigenesis because angiogenesis usually precede metastasis. Although angiogenesis is a normal process in growth and development it is also a fundamental step in the transition of tumors from a dormant state to a malignant one. It was found that PIM-3 is highly expressed both at mRNA and protein levels in endothelial cells and the protein is co-localized at the cellular lamelliopodia focal kinase (FAK), a kinase involved in cellular adhesion and spreading processes. FAK is typically located at structures known as focal adhesions; these are multi-protein structures that link the extracellular matrix to the cytoplasmic cytoskeleton. It is recruited as a participant in focal adhesion dynamics between cells and has a role in motility and cell survival. FAK have also tyrosine kinase activity and originally identified as a substrate for the oncogene protein. After treatment with cytochalasin D which disrupts actin microfilaments, PIM-3 was dispersed from lamelliopodia suggesting strong interaction of PIM-3 with cytoskeleton. Furthermore knockdown of PIM-3 by siRNA had significant effects on endothelial cells migration, proliferation and formation of sprouts. In light of this finding PIM-3 kinase seems to be a new and promising target for novel inhibitors of angiogenesis.

PIM-3 overexpression has been observed in several human cancers, mainly solid tumors like gastrointestinal, colon or liver cancers where expression of PIM-3 seems to be also a poor prognostic marker, however its role in development of pancreatic adenocarcinoma has been studies in more detail (Popivanova *et al.,* 2007; Zheng *et al*., 2008). PIM-3 was found to be expressed in malignant lesions of the pancreas but not in normal pancreatic tissue (Li *et al.,* 2006). In line with this finding, PIM-3 mRNA and protein were constitutively expressed in all examined human pancreatic cancer cell lines. Knock down of the *PIM-1* mRNA levels resulted in apoptosis of the cells, proving essential role of PIM-3 in inhibition of apoptosis in pancreatic cancer cell lines. Further experiments showed that expression of PIM-3 in pancreatic cell lines is controlled by binding of the Ets-1 protein to the 5'-flanking region of human *PIM-3* gene between -249 and -183 bp (Li *et al.,* 2009). Overexpression of Ets-1 transcription factor was able to stimulate transcription and translation of the PIM-3 kinase. These observations indicate that the transcription factor Ets-1 can induce aberrant PIM-3 expression and subsequently prevent apoptosis in human pancreatic cancer cells. Despite the fact that PIM-3 is a kinase of emerging role in cancer development, presented above results implicate how important and diversified roles PIM-3 may play in tumorigenesis and provide rationale for further development of PIM-3 inhibitors for cancer treatment.

CLK kinases belong to Lammer dual specificity kinase subfamily and phosphorylate serines, threonines and tyrosines. The family consists of 4 members (Clk1/Sty and Clk2-4). CLKs are dual-specificity kinases, which have the ability to autophosphorylate themselves at tyrosine residues but phosphorylate their substrates exclusively on serine/threonine residues. These kinases phosphorylate serine- and arginine-rich (SR) proteins of the spliceosomal complex like ASF/SF2, SRp40 and SRp55, critical components of splicesosomes (Soret and Tazi, 2003; Stojdl and Bell, 1999). Alternative splicing is a crucial mechanism for generating protein diversity. Different splice variants of a given protein can display different and even antagonistic biological functions. Therefore, appropriate control of their synthesis is required to assure the complex orchestration of cellular processes within multicellular organisms. Mechanisms that alter the accuracy of either constitutive or alternative splicing could have a profound impact on human pathogenesis, in particular in tumor development and progression (Hagiwara, 2005).

Clk kinases were so far shown to be implicated in regulation of alternative splicing of only few genes like tissue factor, VEGF receptor and PKCbeta II kinase. Apart from VEGF splicing, where Clk seem to have rather beneficial role and act in a anti-angiogenic way leading to formation of anti-angiogenic form of VEGF-b, there is no direct evidence in literature on their role in cancer development. There are however indirect reports showing that they might play a role in such cancers like erythroleukemia. In a study by Garcia-Sacristan from 2005 it was shown that Clk/STY, as well as other members of the family (clk2, clk3 and clk4), are upregulated during HMBA-induced erythroleukemia cell differentiation(Garcia-Sacristan *et al.,* 2005). In a recent article by Jiang *et al.,* it was shown that Akt2, in response to insulin, resulted in phosphorylation of Clk/Sty, which then altered SR protein phosphorylation in concert with Akt2 (Jiang *et al.,* 2009). Apart from its importance in diabetes, the influence of Clk inhibitor on PKC beta splicing can be important in cancer treatment. There is evidence that PKCbeta can contribute in several ways to tumor formation. In addition to direct effects on tumor cells, PKCbeta is involved in tumor host mechanisms such as inflammation and angiogenesis. Elevated expression ofPKCbetaII seems to be an early event in colon cancer development and transgenic overexpression of PKCbetaII in the intestine induces hyper-proliferation and an invasive phenotype in epithelial cells by activating beta-catenin/Apc signaling pathway. A study by Abrams demonstrated that overexpression of the PKCbetaII isoform is a feature of CLL (chronic lymphocytic leukemia) cells and that activity of this enzyme strongly correlates with CLL cell response to BCR engagement.

Benzimidazole derivatives substituted with halogens have been previously described as protein kinase inhibitors of IRAK, CK2, DYRK, HIPK and PIM kinases (WO03/030902 A1; WO2005/092866; Gianoncelli et al., 2009; Pagano et al., 2008 and 2004; Andrzejewska et al., 2003). In an article by Pagano, et. al the art described therein teaches about a class of tetrabromobenzimidazole compounds that are substituted with a group of atoms forming an open chain. Embodiments of the present invention, represented by Formulas A and B, adds the novelty of cyclic substituents that are selected from a group of carbocycles and heterocycles which may be saturated, unsaturated, or aromatic, not taught by the Pagano et al., 2004 (see Scheme 1 and Table 1 from Pagano, et. al. 2004). The present invention described herein, represented inter alia by Formulas A and B, is further distinguished from findings of Pagano, et.al 2008, by notable differences observed in the structure activity relationship. Pagano, et. al, teaches that when the substituent on N-1 is other than hydrogen PIM1 receptor binding activity is relatively weaker as can be deduced from comparison of pairs of compounds like K10 with K15 and K25 with K40 (Pagano et al 2008, see Table S2). Whereas, the inventors of the present invention have inter alia demonstrated that when R1 is, for example, ethyl or isopropyl (compounds A and B) PIM1 activity is equally good or better, and such compounds can inhibit or reverse the growth of cancer cells in-vitro and in vivo. Articles by Battistutta, et. al 2005 and Bortolato, et. al 2007 refer to the same class of tetrabromobenzimidazole compounds as described by Pagano, et. al (2004 and 2008) and do not teach anything new. The single exception is a tetrabromobenzimidazole CK2 inhibitor by Bortolato, et. al, which contains a sulfur linked nitrobenzene substituent, and is not described within the scope of this invention (Battistutta et al 2005; Bortolato et al 2007).

### OBJECTS AND SUMMARY OF THE INVENTION

The major goal of the present invention is to provide novel derivatives of tetrahalogenated benzimidazoles with more potent anticancer activity in comparison to the previously published compounds and improved potency and specificity towards serine/threonine kinases. The invention also reports on other groups of kinases as new kinase targets of tetrahalogenated benzimidazoles, which were not reported previously and which belong to groups of CDK, FLT, HIPK, CLK, PKG, Haspin, MER, TAO, MNK and TRK kinases. These kinases are new targets of tetrahalogenated benzimidazoles that were to the knowledge of the inventors so far not described in the literature. Thus, examples provided by the prior art (like Pagano et al., 2008, Biochem. J) teach that DMAT and TBB when tested at 1 µM do not inhibit CDK2/cyclin A activity (Table 1 in above publication). In contrast, the inventors of the present invention were inter alia able to show that compounds according to the present invention exert more potent activity in inhibiting kinases exemplified but not limited to CDK kinases (as shown in Table 5 of the present application). Inhibition of these novel targets of tetrahalogenated benzimidazoles may contribute to unexpected and improved anticancer activity of these compounds. Moreover, compounds of the present invention exert improved solubility, bioavailability and metabolic stability and are thus very promising for pharmaceutical development and treatment of diseases like cancer and immunological disorders.

It has inter alia been surprisingly found that compounds of this invention and pharmaceutically acceptable compositions thereof are effective inhibitors of the PIM kinase family, but also other kinases like the CDK, FLT, HIPK, CLK, PKG, Haspin, MER, TAO, MNK and TRK kinases. These compounds exert also improved cytotoxic activity in vitro when tested on a panel of neoplastic cell lines. In contrast to tetrabromobenzimidazole derivatives published in the prior art, which in general did not reach the ED₅₀ values below 10 µM for more than 2 or 3 neoplastic cell lines, compounds presented in the application show better cytotoxicity defined as ED₅₀ values below 10 µM in at least five different neoplastic cell lines of both hematological and solid tumor origin. This feature will inter alia allow broader therapeutic indication of the compounds from the current application to treat e.g. various cancer types. In addition, one of the technical features that renders further pharmaceutical development and medical use of the tetrabromobenzimidazoles is their very low solubility in physiological solutions and pH above 7, and this feature could also be improved for compounds according to the present invention.

Subject of the present invention are inter alia compounds as claimed in independent claim 1.

In a preferred embodiment, n is 1 to form a 5-membered carbocycle or heterocycle, but not aromatic.

In another preferred embodiment, n is 2 to form a 6-membered carbocycle or heterocycle but not aromatic.

In still another preferred embodiment, n is selected from 1, 2, or 3 to form a 5-, 6-, or 7-membered carbocycle heterocycle but not aromatic.

In another preferred embodiment, Z¹ and Z² may be taken together independently to form fused rings that are 5-, 6-, or 7-membered carbocycles or heterocycles, which may be saturated or unsaturated or aromatic and which may be substituted as set out above. In still another preferred embodiment, Z¹ and Z² may be taken together to form a second fused ring that is a 5-, 6-, or 7-membered carbocycle or heterocycle, which may be saturated or unsaturated or aromatic and which may be substituted as set out above.

In another preferred embodiment, R_{A} is independently selected at each occurrence from the group consisting of H, amino, hydroxyl, aminoalkyl, alkoxy, carboxylic acid, carboxyester, carboxamide, carbamate, sulfonic acid, sulfonamide, trifluoromethyl and preferably monocyclic 5- to 8-membered carbocyclic and heterocyclic groups, which are saturated, unsaturated, or aromatic and which may be substituted as set out above.

In the following, preferred embodiments of the present invention referring to particularly preferred compounds of formula (A) as shown above are listed.

Thus, in a preferred embodiment, the present invention refers to compounds of formula (A) wherein Z⁰, Z¹, Z² and n are selected such that a substituted or unsubstituted 6-membered carbocycle or heterocycle is formed, which is saturated or unsaturated but not aromatic.

Preferably, said substituted or unsubstituted carbocycle or heterocycle is saturated.

In a further preferred embodiment, the present invention refers to compounds of formula (A) wherein Z⁰, Z¹, Z² and n are selected such that a substituted or unsubstituted 6-membered heterocycle is formed, which is saturated or unsaturated but not aromatic.

Preferably, said substituted or unsubstituted heterocycle is saturated. More preferably, said heterocycle comprises two N-heteroatoms. Even more preferably, said heterocycle is unsubstituted or substituted by a single substituent only, which preferably corresponds to R⁴.

In a further preferred embodiment, the present invention refers to compounds of formula (A) wherein Z⁰, Z¹, Z² and n are selected such that a substituted or unsubstituted 6-membered heterocycle corresponding to substituted or unsubstituted piperazine is formed. Thus, in such an embodiment, Z⁰ is N, Z¹ is CHR³, n is 2, Z²(1) is CHR³ and Z²(2) is NR⁴.

In all the above mentioned embodiments it can be preferred that R³ is at each occurrence H or at at least one occurrence -Y¹(C₁₋₆alkyl)R_{A}. If R³ is in said embodiments at at least one occurrence -Y¹(C₁₋₆alkyl)R_{A}, it can be preferred that-Y¹ is absent and that R_{A} is a 6-membered substituted or unsubstituted carbocyclic or heterocyclic group, preferably a substituted or unsubstituted piperazine. It can further be preferred that R⁴ is H or -Y¹(C₁₋₆alkyl)R_{A}. If R⁴ is in said embodiments -Y¹(C₁₋₆alkyl)R_{A}, it can be preferred that -Y¹ is absent and that R_{A} is a 6-membered substituted or unsubstituted carbocyclic or heterocyclic group, preferably a substituted or unsubstituted piperazine, more preferably an unsubstituted piperazine. Especially preferred embodiments of the above mentioned embodiments refer to compounds, wherein both R³ and R⁴ are H at all occurrences.

It can further be preferred in all the above embodiments that X¹ is at all occurrences selected from either F, Cl or Br, wherein Br is especially preferred.

It can also be preferred in all the above embodiments that R¹ is selected from H, methyl, (C₂₋₆alkyl)R_{A}, and saturated 5- to 6-membered carbocyclic groups. H can be particularly preferred. If R¹ is in said embodiments (C₂₋₆alkyl)R_{A,}, it can be particularly preferred that R_{A} is H and that said alkyl is selected from a substituted or unsubstituted ethyl, propyl, isopropyl and butyl, wherein said alkyl is preferably unsubstituted and most preferably unsubstituted ethyl or isopropyl.

Further objects of the present invention inter alia refer to pharmaceutical compositions comprising compounds (A) according to claim 1 . Said further objects will be referred to in the detailed description below.

### DESCRIPTION OF THE FIGURES

Figure 1 shows the inhibition of biomarkers as indicated on the left side (Piml as expression control, p-4EBP1, c-myc and as negative control tubulin) in MV411 cells after 4h and 24h treatment with compound 20 in the concentrations as indicated above each lane of the Western blot, S corresponds to Sunitinib. Standard antibodies against the indicated biomarkers were used in the Western blots.
Figure 2 shows the MV411 tumor growth progression after treatment with compounds 20, 359, and 416. Details of the experiment are described in example 13.

### DETAILED DESCRIPTION OF THE INVENTION

The following is a list of definitions for terms used herein.

"Alkyl" is a saturated hydrocarbon chain having 1 to 15 carbon atoms, preferably 1 to 10, more preferably 1 to 4 carbon atoms. It is, however, clear to the skilled person that an indication of the number of carbon atoms in connection with the term "alkyl" refers to a hydrocarbon chain having the indicated number of carbon atoms; thus, e.g."C₁₋₆alkyl" means that said hydrocarbon chain has 1 to 6 carbon atoms. "Alkenyl" is a hydrocarbon chain having at least one (preferably only one) carbon-carbon double bond and having 2 to 15 carbon atoms, preferably 2 to 10, more preferably 2 to 4 carbon atoms. "Alkynyl" is a hydrocarbon chain having at least one (preferably only one) carbon-carbon triple bond and having 2 to 15 carbon atoms, preferably 2 to 10, more preferably 2 to 4 carbon atoms. Alkyl, alkenyl and alkynyl chains (referred to collectively as "hydrocarbon chains") may be straight or branched and may be unsubstituted or substituted. Preferred branched alkyl, alkenyl and alkynyl chains have one or two branches, preferably one branch. Preferred chains are alkyl. Alkyl, alkenyl and alkynyl hydrocarbon chains each may be unsubstituted or substituted with from 1 to 4 substituents; when substituted, preferred chains are mono-, di-, or tri-substituted. Alkyl, alkenyl and alkynyl hydrocarbon chains each may be substituted with halo, hydroxy, aryloxy (e.g. phenoxy), heteroaryloxy, acyloxy (e.g. acetoxy), carboxy, aryl (e.g. phenyl), heteroaryl, cycloalkyl, heterocycloalkyl, spirocycle, amino, amido, acylamino, keto, thioketo, cyano, or any combination thereof. Preferred hydrocarbon groups include methyl, ethyl, propyl, isopropyl, butyl, vinyl, allyl, and butenyl.

Also, as referred to herein, a "lower" alkyl, alkenyl or alkynyl moiety (e.g. "lower alkyl") is a chain comprised of 1 to 6, preferably from 1 to 4, carbon atoms in the case of alkyl and 2 to 6, preferably 2 to 4, carbon atoms in the case of alkenyl and alkynyl.

"Aryl" is an aromatic hydrocarbon ring. Aryl rings are monocyclic or fused bicyclic ring systems. Monocyclic aryl rings contain 6 carbon atoms in the ring. Monocyclic aryl rings are also referred to as phenyl rings. Bicyclic aryl rings contain from 8 to 17 carbon atoms, preferably 9 to 12 carbon atoms, in the ring. Bicyclic aryl rings include ring systems wherein one ring is aryl and the other ring is aryl, cycloalkyl, or heterocycloalkyl. Preferred bicyclic aryl rings comprise 5-, 6- or 7-membered rings fused to 5-, 6-, or 7-membered rings. Aryl rings may be unsubstituted or substituted with from 1 to 4 substituents on the ring. Aryl may be substituted with halo, cyano, nitro, hydroxy, carboxy, amino, acylamino, alkyl, heteroalkyl, haloalkyl, phenyl, aryloxy, alkoxy, heteroalkyloxy, carbamyl, haloalkyl, methylenedioxy, heteroaryloxy, or any combination thereof. Preferred aryl rings include naphthyl, tolyl, xylyl, and phenyl. The most preferred aryl ring radical is phenyl.

"Cycloalkyl" is a saturated hydrocarbon ring. Cycloalkyl rings are not aromatic. Cycloalkyl rings are monocyclic, or are fused, spiro, or bridged bicyclic ring systems. Monocyclic cycloalkyl rings contain from about 3 to about 9 carbon atoms, preferably from 3 to 7 carbon atoms, in the ring. Bicyclic cycloalkyl rings contain from 7 to 17 carbon atoms, preferably from 7 to 12 carbon atoms, in the ring. Preferred bicyclic cycloalkyl rings comprise 4-, 5-, 6- or 7-membered rings fused to 5-, 6-, or 7-membered rings. Cycloalkyl rings may be unsubstituted or substituted with from 1 to 4 substituents on the ring. Cycloalkyl may be substituted with halo, cyano, alkyl, heteroalkyl, haloalkyl, phenyl, keto, hydroxy, carboxy, amino, acylamino, aryloxy, heteroaryloxy, or any combination thereof. Preferred cycloalkyl rings include cyclopropyl, cyclopentyl, and cyclohexyl.

"Heteroatom" is a nitrogen, sulfur, or oxygen atom. Groups containing more than one heteroatom may contain different heteroatoms.

"Heteroalkyl" is a saturated chain containing carbon and at least one heteroatom, wherein no two heteroatoms are adjacent. Heteroalkyl chains contain from 2 to 15 member atoms (carbon and heteroatoms) in the chain, preferably 2 to 10, more preferably 2 to 5. For example, alkoxy (i.e. -O-alkyl or -O-heteroalkyl) radicals are included in heteroalkyl. Heteroalkyl chains may be straight or branched. Preferred branched heteroalkyl have one or two branches, preferably one branch. Heteroalkyl chains may be unsubstituted or substituted with from 1 to 4 substituents.

Preferred substituted heteroalkyl are mono-, di-, or tri-substituted. Heteroalkyl may be substituted with lower alkyl, haloalkyl, halo, hydroxy, aryloxy, heteroaryloxy, acyloxy, carboxy, monocyclic aryl, heteroaryl, cycloalkyl, heterocycloalkyl, spirocycle, amino, acylamino, amido, keto, thioketo, cyano, or any combination thereof.

"Heteroaryl" is an aromatic ring containing carbon atoms and from 1 to about 6 heteroatoms in the ring. Heteroaryl rings are monocyclic or fused bicyclic ring systems. Monocyclic heteroaryl rings contain from about 5 to about 9 member atoms (carbon and heteroatoms), preferably 5 or 6 member atoms, in the ring. Bicyclic heteroaryl rings contain from 8 to 17 member atoms, preferably 8 to 12 member 60 atoms, in the ring. Bicyclic heteroaryl rings include ring systems wherein one ring is heteroaryl and the other ring is aryl, heteroaryl, cycloalkyl, or heterocycloalkyl. Preferred bicyclic heteroaryl ring systems comprise 5-, 6- or 7-membered rings fused to 5-, 6-, or 7-membered rings. Heteroaryl rings may be unsubstituted or substituted with from 1 to 4 substituents on the ring. Heteroaryl may be substituted with halo, cyano, nitro, hydroxyl, carboxy, amino, acylamino, alkyl, heteroalkyl, phenyl, alkoxy, aryloxy, heteroaryloxy, or any combination thereof.

Preferred heteroaryl rings include, but are not limited to, the following:

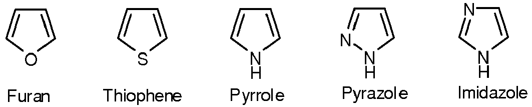

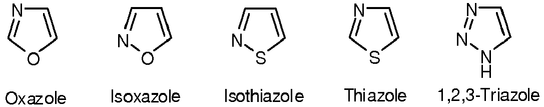

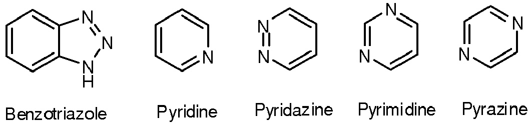

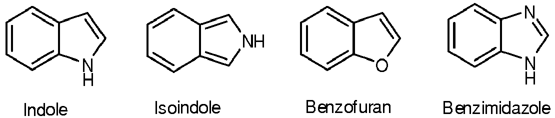

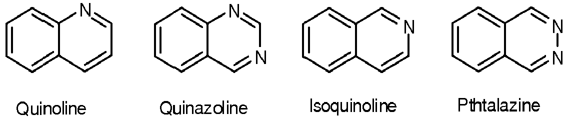

"Halogen" is fluorine, chlorine, or bromine.

"Aminoalkyl" is -NH(C1-6alkyl), or -N(Cl-6alkyl)₂ or a quaternized amine, i.e.-N(C1-6alkyl)₃.

"Alkoxy" is -O(C1-6alkyl).

"Carboxylic acid" is -C(O)OH.

"Carboxy" is -C(O)O-.

"Carboxyester" is -C(O)O(C1-6alkyl).

"Carboxamide" is -C(O)NH-, -C(O)N(C1-6alkyl)-.

"Carbamate" is -OC(O)NH-, -OC(O)N(C1-6alkyl)-.

"Sulfonic acid" is -S(O)₂OH.

"Sulfonamide" is -S(O)₂NH-, or -S(O)₂N(C1-6alkyl)-.

"Oxo" is -O-.

"Carbonyl" is -C(O)-.

"Aminoalkylamine" is -NH(C1-6alkyl)NH(C1-6alkyl), or -NH(C1-6alkyl)N(C1-6alkyl)₂.

"Aminoalkylalkoxy" is -NH(C1-6alkyl)O(C1-6alkyl),-O(C1-6alkyl)NH(C1-6alkyl),-O(C1-6alkyl)N(C1-6alkyl)₂.

"Amidine" is -C(NH)NH₂.

"Nitrile" is -CN.

"Sulfone" is an optionally cyclic structure of -(C1-6alkyl)S(O)₂(C1-6alkyl).

"Heterocycloalkyl" is a saturated ring containing carbon atoms and from 1 to about 4 (preferably 1 to 3) heteroatoms in the ring. Heterocycloalkyl rings are not aromatic. Heterocycloalkyl rings are monocyclic, or are fused, bridged, or spiro bicyclic ring systems. Monocyclic heterocycloalkyl rings contain from about 3 to about 9 member atoms (carbon and heteroatoms), preferably from 5 to 7 member atoms, in the ring. Bicyclic heterocycloalkyl rings contain from 7 to 17 member atoms, preferably 7 to 12 member atoms, in the ring. Bicyclic heterocycloalkyl rings contain from about 7 to about 17 ring atoms, preferably from 7 to 12 ring atoms. Bicyclic heterocycloalkyl rings may be fused, spiro, or bridged ring systems. Preferred bicyclic heterocycloalkyl rings comprise 5-, 6- or 7-membered rings fused to 5-, 6-, or 7-membered rings. Heterocycloalkyl rings may be unsubstituted or substituted with from 1 to 4 substituents on the ring. Heterocycloalkyl may be substituted with halo, cyano, hydroxy, carboxy, keto, thioketo, amino, acylamino, acyl, amido, alkyl, heteroalkyl, haloalkyl, phenyl, alkoxy, aryloxy or any combination thereof. Preferred substituents on heterocycloalkyl include halo and haloalkyl. Preferred heterocycloalkyl rings include, but are not limited to, the following:

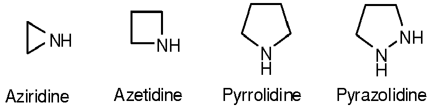

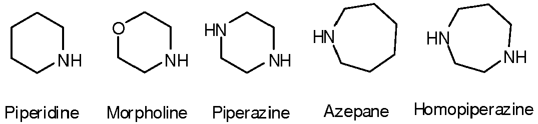

While alkyl, heteroalkyl, cycloalkyl, and heterocycloalkyl groups may be substituted with hydroxy, amino, and amido groups as stated above, the following are not envisioned in the invention:
Enols (OH attached to a carbon bearing a double bond).
Amino groups attached to a carbon bearing a double bond (except for vinylogous amides).

More than one hydroxy, amino, or amido attached to a single carbon (except where two nitrogen atoms are attached to a single carbon atom and all three atoms are member atoms within a heterocycloalkyl ring).

Hydroxy, amino, or amido attached to a carbon that also has a heteroatom attached to it.

Hydroxy, amino, or amido attached to a carbon that also has a halogen attached to it. Some of the compounds disclosed herein may contain one or more asymmetric centers and may thus lead to enantiomers, diastereomers, and other stereoisomeric forms.

The present invention is also meant to encompass all such possible forms as well as their racemic and resolved forms and mixtures thereof, unless specified otherwise. When the compounds described herein contain olefinic double bonds or other centers of geometric asymmetry, and unless specified otherwise, it is intended to include both E and Z geometric isomers. All tautomers are intended to be encompassed by the present invention as well.

As used herein, the term "stereoisomers" is a general term for all isomers of individual molecules that differ only in the orientation of their atoms in space. It includes enantiomers and isomers of compounds with more than one chiral center that are not mirror images of one another (diastereomers). The term "chiral center" refers to a carbon atom to which four different groups are attached. The term "enantiomer" or "enantiomeric" refers to a molecule that is nonsuperimposeable on its mirror image and hence optically active wherein the enantiomer rotates the plane of polarized light in one direction and its mirror image rotates the plane of polarized light in the opposite direction. The term "racemic" refers to a mixture of equal parts of enantiomers and which is optically inactive. The term "resolution" refers to the separation or concentration or depletion of one of the two enantiomeric forms of a molecule.

As used in the specification and the claims, the singular forms of "a" and "an" also include the corresponding plurals unless the context clearly dictates otherwise.

The terms "about" and "approximately" in the context of the present invention denotes an interval of accuracy that a person skilled in the art will understand to still ensure the technical effect of the feature in question. The term typically indicates a deviation from the indicated numerical value of ±10% and preferably ±5%.

It needs to be understood that the term "comprising" is not limiting. For the purposes of the present invention, the term "consisting of" is considered to be a preferred embodiment of the term "comprising of". If hereinafter a group is defined to comprise at least a certain number of embodiments, this is also meant to encompass a group which preferably consists of these embodiments only.

Preferably, the compound is selected from Table 1:

**Table 1. Example list I (Formula A)**

| **Compound number** | **Name/Properties** | **Structure** |
|---|---|---|
| **18** | 4,5,6,7-tetrabromo-2-(pyrrolidin-1-yl)-1*H-*benzo[*d*]imidazole; C₁₁H₉Br₄N₃ (MW 502.82) General procedure A; m/z 503.7 [M+H]⁺; RT= 7.5 min | 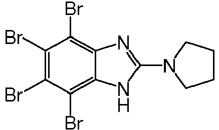 |
| **19** | 4,5,6,7-tetrabromo-2-morpholino-1H-benzo[*d*]imidazole; C₁₁H₉Br₄N₃O (MW 518.82) General procedure A; m/z 519.7 [M+H]⁺; RT= 12.5 min | 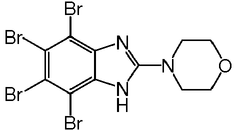 |
| **20** | 4,5,6,7-tetrabromo-2-(piperazin-1-yl)-1*H-*benzo[*d*]imidazole; C₁₁H₁₀Br₄N₄ (MW 517.84) General procedure A; m/z 518.6 [M+H]⁺; RT= 5.4 min | 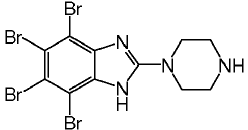 |
| **21** | 4,5,6,7-tetrabromo-2-(4-methylpiperazin-1-yl)-1*H-*benzo[*d*]imidazole; C₁₂H₁₂Br₄N₄(MW 531.87) General procedure A; m/z 532.7 [M+H]⁺; RT= 5.5 min | 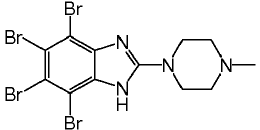 |
| **75** | 3-(4-(4,5,6,7-tetrabromo-1*H-*benzo[*d*]imidazol-2-yl)piperazin-1-yl)-N,N-dimethylpropan-1-amine; C₁₆H₂₁Br₄N₅ (MW 602.99) General procedure A; m/z 603.3[M+H]⁺; RT= 3.1 min | 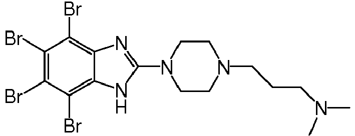 |
| **76** | 4,5,6,7-tetrabromo-2-(4-(2-(piperidin-1-yl)ethyl)piperazin-1-yl)-1*H-*benzo[*d*]imidazole; C₁₈H₂₃Br₄N₅ (MW 629.02) General procedure A; m/z 629.8 [M+H]⁺; | 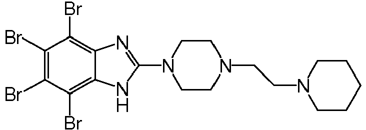 |
| **103** | 3-(4-(4,5,6,7-tetrabromo-1*H-*benzo[*d*]imidazol-2-yl)piperazin-1-yl)-N,N,N-trimethylpropan-1-ammonium bromide; C₁₇H₂₄Br₅N₅ (MW 697.93) General procedure B; m/z 617.9 [M-H]⁻; RT= 2.3 min | 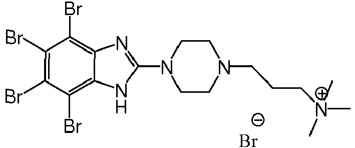 |
| **242** | 4,5,6,7-tetrabromo-2-(indolin-1-yl)-1*H*-benzo[*d*]imidazole; C₁₅H₉Br₄N₃ (MW 550.87) General procedure A; m/z 551.7[M+H]⁺; RT= 23.4 min | 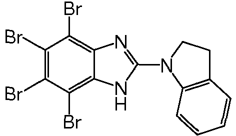 |
| **256** | 4,5,6,7-tetrabromo-2-(4-methyl-1,4-diazepan-1-yl)-1*H*-benzo[*d*]imidazole; C₁₃H₁₄Br₄N₄ (545.89) General procedure A; m/z 546.7 [M+H]⁺; RT= 5.6 min | 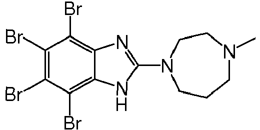 |
| **260** | 4,5,6,7-tetrabromo-2-(1,4-diazepan-1-yl)-1*H-*benzo[*d*]imidazole; C₁₂H₁₂Br₄N₄ (531.87) General procedure A; m/z 532.7 [M+H]⁺; RT= 5.6 min | 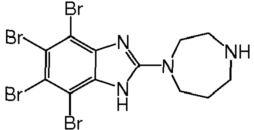 |
| **262** | 4,5,6,7-tetrabromo-2-(3-((2-methyl-1*H*-imidazol-1-yl)methyl)piperidin-1-yl)-1*H-*benzo[*d*]imidazole; C₁₇H₁₇Br₄N₅ (610.97) General procedure A; m/z 611.8 [M+H]⁺; RT= 6.7 min | 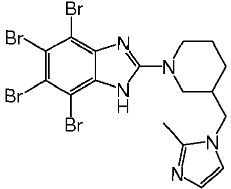 |
| **266** | 4,5,6,7-tetrabromo-2-(3-((4-(pyridin-2-yl)piperazin-1-yl)methyl)piperidin-1-yl)-1*H-*benzo[*d*]imidazole C₂₂H₂₄Br₄N₆ (692.07) General procedure A; m/z 692.9 [M+H]⁺; RT= 6.6 min | 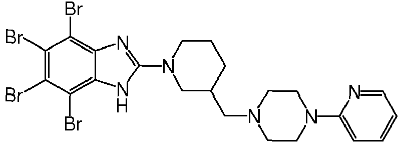 |
| **301** | 4,5,6,7-tetrabromo-2-(4-(1-[1-(1,1-dioxidotetrahydro-3-thienyl)-3,5-dimethyl-1*H-*pyrazol-4-yl]piperazin-1-yl-1*H*-benzo[*d*]imidazole; C₂₀H₂₂Br₄N₆O₂S (730.11) General procedure A; m/z 730.8 [M+H]⁺; RT= 17.6 min | 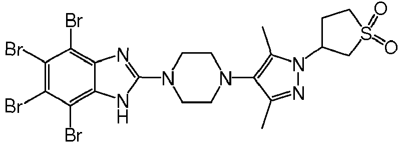 |
| **305** | 1-(4,5,6,7-tetrabromo-1*H-*benzo[*d*]imidazol-2-yl)-N-(5-methylthiazol-2-yl)piperidine-4-carboxamide; C₁₇H₁₅Br₄N₅OS (657.02) General procedure A; m/z | 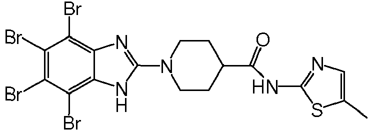 |
| | 657.8 [M+H]⁺; RT= 16.3 min | |
| **343** | 2-(4-(4,5,6,7-tetrabromo-1*H-*benzo[*d*]imidazol-2-yl)piperazin-1-yl)-N,N-dimethylethanamine; C₁₅H₁₉Br₄N₅ (588.96) General procedure A; m/z 589.9 [M+H]⁺; RT= 5.4 min | 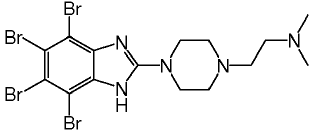 |
| **350** | 2-(4,5,6,7-tetrabromo-1*H-*benzo[*d*]imidazol-2-yl)-2,5-diaza-bicyclo[3.2.2]nonane; C₁₄H₁₄Br₄N₄ (557.9) General procedure A; m/z 558.9 [M+H]⁺; RT= 6.0 min | 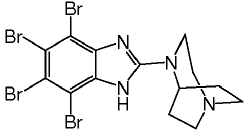 |
| **353** | 4,5,6,7-tetrabromo-2-((3aR,7aS)-octahydroindol-1-yl)-1*H*-benzo[*d*]imidazole; C₁₄H₁₂Br₄N₄ (555.89) General procedure A; m/z 577.8 [M+23]⁺; RT= 12.7 min | 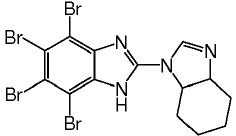 |
| **359** | 4,5,6,7-tetrabromo-1-isopropyl-2-(piperazin-1-yl)-1*H*-benzo[*d*]imidazole; C₁₄H₁₆Br₄N₄ (559.92) General procedure A; m/z 560.7 [M+H]⁺; RT= 7.5 min | 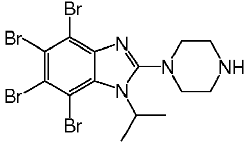 |
| **364** | 4,5,6,7-tetrabromo-2-((2S,5R)-2,5-dimethylpiperazin-1-yl)-1*H-*benzo[*d*]imidazole; C₁₃H₁₄Br₄N₄ (545.89) General procedure A; m/z 546.7 [M+H]⁺; RT= 6.3 min | 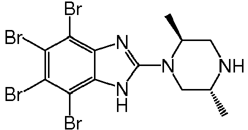 |
| **367** | 1-(4,5,6,7-tetrabromo-1*H-*benzo[*d*]imidazol-2-yl)-1,2,3,4-tetrahydroquinoxaline; C₁₅H₁₀Br₄N₄ (565.88) General procedure A; m/z 566.6 [M+H]⁺; RT= 19.5 min | 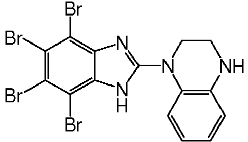 |
| **374** | 1-benzyl-4,5,6,7-tetrabromo-2-(piperazin-1-yl)-1*H-*benzo[*d*]imidazole; C₁₈H₁₆Br₄N₄ (607.96) General procedure A; m/z 608.6 [M+H]⁺; RT= 8.2 min | 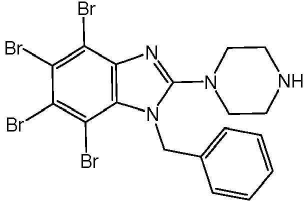 |
| **376** | ethyl 4-(4,5,6,7-tetrabromo-1*H*-benzo[*d*]imidazol-2-yl)piperazine-1-carboxylate; C₁₅H₁₆Br₄N₄O₂ (603.93) General procedure C; m/z 604.7[M+H]⁺; RT= 9.2 min | 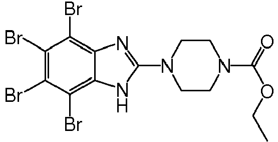 |
| **377** | ethyl 2-(4-(ethoxycarbonyl)piperazin-1-yl)-4,5,6,7-tetrabromo-1*H-*benzo[*d*]imidazole-1-carboxylate; C₁₉H₂₂Br₄N₄O₄ (690.02) General procedure C; m/z 690.8 [M+H]⁺; RT= 11.6 min | 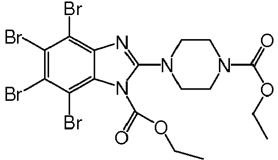 |
| **378** | 1-(4,5,6,7-tetrabromo-1*H-*benzo[*d*]imidazol-2-yl)-N,N-dimethylpiperidin-3-amine; C₁₄H₁₆Br₄N₄ (559.92) General procedure A; m/z 560.8 [M+H]⁺; RT= 6.1 min | 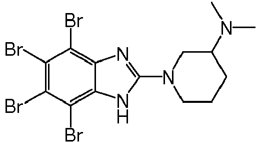 |
| **383** | 3-(4-(4,5,6,7-tetrabromo-1*H-*benzo[*d*]imidazol-2-yl)piperazin-1-yl)propan-1-ol; C₁₄H₁₆Br₄N₄O₄ (575.92) General procedure A; m/z 576.7 [M+H]⁺; RT= 5.5 min | 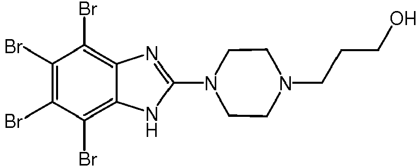 |
| **384** | 2-(4-(4,5,6,7-tetrabromo-1*H-*benzo[*d*]imidazol-2-yl)piperazin-1-yl)-3-methylbutanenitrile; C₁₆H₁₇Br₄N₅ (MW 598.96) General procedure A; m/z 599.8 [M+H]⁺; RT= 20.2 min | 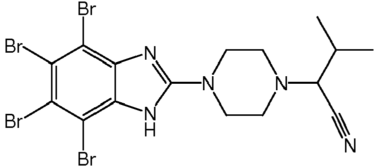 |
| **385** | 4,5,6,7-tetrabromo-2-(4-(4-methylpiperazin-1-yl)piperidin-1-yl)-1*H-*benzo[*d*]imidazole; C₁₇H₂₁Br₄N₅(MW 615.0) General procedure A; m/z 615.7 [M+H]⁺; RT= 5.3 min | 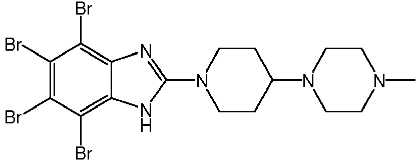 |
| **386** | 4,5,6,7-tetrabromo-2-(4-(piperidin-4-yl)piperidin-1-yl)-1*H*-benzo[*d*]imidazole; C₁₇H₂₀Br₄N₄ (599.98) General procedure A; m/z 600.8 [M+H]⁺; RT= 6.9 min | 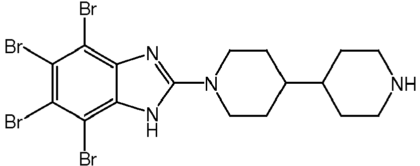 |
| **387** | 2-(4-((H-imidazo[1,2-*a*]pyridin-2-yl)methyl)piperazin-1-yl)-4,5,6,7-tetrabromo-1*H-*benzo[*d*]imidazole; C₁₉H₁₆Br₄N₆ (647.99) General procedure A; m/z 650.8 [M+H]⁺; RT= 6.2 min | 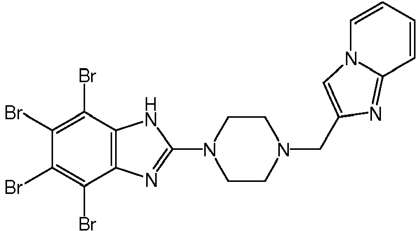 |
| **389** | 4,5,6,7-tetrabromo-2-(4-((2-(4-fluorophenyl)thiazol-4-yl)methyl)piperazin-1-yl)-1*H-*benzo[*d*]imidazole; C₂₁H₁₆Br₄FN₅S (709.06) General procedure A; m/z 709.7 [M+H]⁺; RT= 9.8 min | 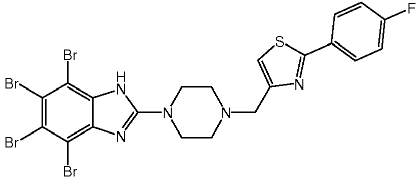 |
| **390** | 4,5,6,7-tetrabromo-2-(4-((3-p-tolyl-1,2,4-oxadiazol-5-yl)methyl)piperazin-1-yl)-1*H-*benzo[*d*]imidazole; C₂₁H₁₈Br₄N₆O (690.02) General procedure; m/z 690.7 [M+H]⁺; RT= 21.3 min | 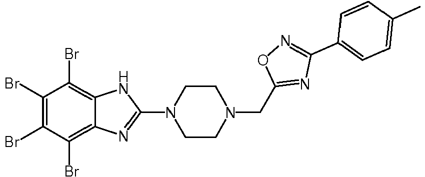 |
| **392** | 4-(4,5,6,7-tetrabromo-1*H-*benzo[*d*]imidazol-2-yl)piperazine-1-carboxamidine; C₁₂H₁₂Br₄N₆ (559.88) General procedure B; m/z 560.8 [M+H]⁺; RT= 5.9 min | 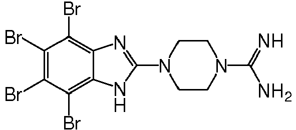 |
| **393** | 2-(4-(4,5,6,7-tetrabromo-1*H-*benzo[*d*]imidazol-2-yl)piperazin-1-yl)acetic acid; C₁₃H₁₂Br₄N₄O₂ (575.88) General procedure C; m/z 576.8 [M+H]⁺; RT= 8.0 min | 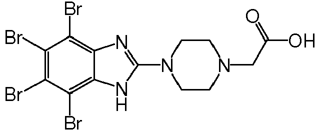 |
| **394** | 2-(4-(3-methoxybenzyl)piperazin-1-yl)-4,5,6,7-tetrabromo-1*H-*benzo[*d*]imidazole; C₁₉H₁₈Br₄N₄O (637.99) General procedure A; m/z 638.8 [M+H]⁺; RT= 8.2 min | 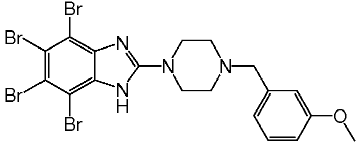 |
| **414** | 4,5,6,7-tetrabromo-2-(4-((2-methyl-1*H*-imidazo[1,2-*a*]pyridin-3-yl)methyl)piperazin-1-yl)-1*H-*benzo[*d*]imidazole; C₂₀H₁₈Br₄N₆ (662.01) General procedure A; m/z 662.7 [M+H]⁺; RT= 6.7 min | 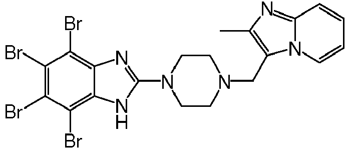 |
| **415** | 4,5,6,7-tetrabromo-1-methyl-2-(piperazin-1-yl)-1*H-*benzo[*d*]imidazole; C₁₂H₁₂Br₄N₄ (531.87) General procedure A; m/z 532.80 [M+H]⁺; RT= 5.9 min | 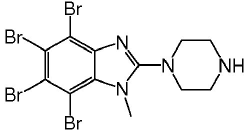 |
| **416** | 4,5,6,7-tetrabromo-1-ethyl-2-(piperazin-1-yl)-1*H-*benzo[*d*]imidazole; C₁₃H₁₄Br₄N₄ (545.89) General procedure A; m/z 546.7 [M+H]⁺; RT= 6.8 min | 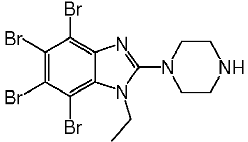 |
| **417** | 4,5,6,7-tetrabromo-2-(piperazin-1-yl)-1-propyl-1*H-*benzo[*d*]imidazole; C₁₄H₁₆Br₄N₄ (559.92) General procedure A; m/z 560.8 [M+H]⁺; RT= 7.7 min | 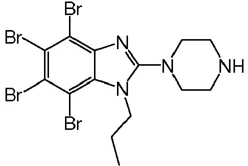 |
| **421** | [4-[4,5,6,7-tetrabromo-1-(carboxymethyl)-1*H-*benzo[*d*]imidazol-2-yl]piperazin-1-yl]acetic acid C₁₅H₁₄Br₄N₄O₄ (633.91). General procedure C; m/z 634.8 [M+H]⁺; RT= 8.1 min | 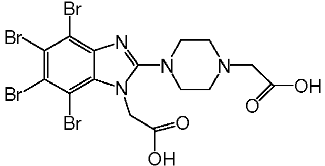 |
| **436** | 4,5,6,7-tetrabromo-2-(4-(2-(piperazin-1-yl)ethyl)piperazin-1-yl)-1*H-*benzo[*d*]imidazole; C₁₇H₂₂Br₄N₆ (630.01) General procedure A; m/z 630.9 [M+H]⁺; RT= 3.6 min | 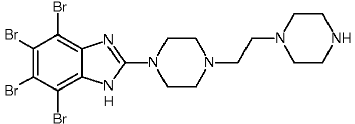 |
| **441** | 4,5,6,7-tetrabromo-1-ethyl-2-(4-(2-(piperazin-1-yl)ethyl)piperazin-1-yl)-1*H-*benzo[*d*]imidazole; C₁₉H₂₆Br₄N₆ (658.07) General procedure A; m/z 658.9 [M+H]⁺; RT=4.4 min | 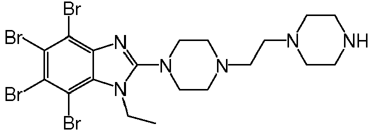 |
| **450** | 4,5,6,7-tetrabromo-1-ethyl-2-(4-(3-morpholinopropyl)piperazin-1-yl)-1*H*-benzo[*d*]imidazole; C₂₀H₂₇Br₄N₅O (673.08) General procedure A; m/z 673.9 [M+H]⁺; RT= 4.0 min | 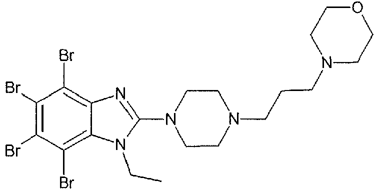 |
| **451** | 4,5,6,7-tetrabromo-2-(4-(3-(piperazin-1-yl)propyl)piperazin-1-yl)-1*H-*benzo[*d*]imidazole; C₁₈H₂₄Br₄N₆ (644.04) General procedure A; m/z 644.9 [M+H]⁺; RT= 2.9 min | 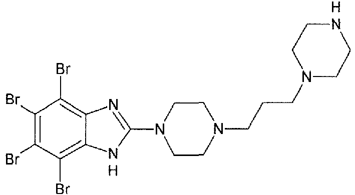 |

Preferably, a pharmaceutically acceptable salt is selected from the group consisting of hydrochloride, hydrobromide, hydroiodide, nitrate, sulfate, bisulfate, phosphate, acid phosphate, isonicotinate, acetate, lactate, salicylate, citrate, tartrate, pantothenate, bitartrate, ascorbate, succinate, maleate, gentisinate, fumarate, gluconate, glucuronate, saccharate, formate, benzoate, glutamate, methanesulfonate, ethanesulfonate, benzensulfonate, p-toluenesulfonate and pamoate and the like.

A "pharmaceutically-acceptable salt" is a cationic salt formed at any acidic (e.g. carboxylic acid) group, or an anionic salt formed at any basic (e.g. amino) group. Many such salts are known in the art, as described in World Patent Publication 87/05297, Johnston et al., published Sep. 11, 1987. Preferred cationic salts include the alkali metal salts (such as sodium and potassium), and alkaline earth metal salts (such as magnesium and calcium) and organic salts. Preferred anionic salts include the halides (such as chloride salts), sulfonates, carboxylates, phosphates, and the like.

Such salts are well understood by the skilled artisan, and the skilled artisan is able to prepare any number of salts given the knowledge in the art. Furthermore, it is recognized that the skilled artisan may prefer one salt over another for reasons of solubility, stability, formulation ease and the like.

The next subject of invention is a pharmaceutical composition, comprising a therapeutically effective amount of at least one compound selected from the group consisting of compounds as described above and optionally comprising a therapeutic agent selected from a chemotherapeutic or anti-proliferative agent, an immunomodulatory or immunosuppressive agent, or an anti-inflammatory agent.

The term "composition" is intended to encompass a product comprising the specified ingredients in the specified amounts, as well as any product which results, directly or indirectly, from combinations of the specified ingredients in the specified amounts. The active agents of the invention are used, alone or in combination with one or more additional active ingredients, to formulate pharmaceutical compositions of the invention. A pharmaceutical composition of the invention comprises an effective amount of at least one active agent in accordance with the invention. As known in pharmaceutical technology, at least one pharmaceutically acceptable carrier may be comprised in embodiments of pharmaceutical compositions according to this invention. A carrier may also be denoted as excipient in the following.

Preferably, the said composition optionally comprises a therapeutic agent selected from a chemotherapeutic or anti-proliferative agent, an immunomodulatory or immunosuppressive agent, or an anti-inflammatory agent.

Preferably, the therapeutically effective amount provided in the treatment is administered in an amount of about 0,01 to 1,000 mg/kg at least once a day for the duration of the treatment.

The term "therapeutically effective amount" as used herein, means that amount of active compound or pharmaceutical agent that elicits the biological or medicinal response in a tissue system, animal or human that is being sought by a researcher, veterinarian, medical doctor or other clinician, which includes alleviation of the symptoms of a disease or disorder. When referring to modulating the target receptor, a "therapeutically effective amount" means an amount sufficient to at least affect the activity of such receptor. Measuring the activity of the target kinase may be performed by routine analytical methods. Target kinase modulation is useful in a variety of settings, including assays. In addition, effective amounts or doses of the active agents of the present invention may be ascertained by routine methods such as modeling, dose escalation studies or clinical trials, and by taking into consideration routine factors, e.g., the mode or route of administration or drug delivery, the pharmacokinetics of the agent, the severity and course of the disease, disorder, or condition, the subject's previous or ongoing therapy, the subject's health status and response to drugs, and the judgment of the treating physician.

The amount varies according to the size, age and response pattern of the patient, the severity of the disorders, the judgment of the attending physician and the like.

Preferably, the said composition is administered parenterally, vaginally, rectally, transdermally, orally or through the otolaryngologal sphere.

Preferably, it further comprises a pharmaceutically acceptable carrier selected from the group consisting of flavoring agents, sweetener, lubricants, solubilizers, suspending agents, fillers, glidants, compression aides, binders, tablet-disintegrating agents, effervescing agent, wetting agent encapsulating materials, dyestuff, and mixtures thereof wherein carrier is chosen from solid or liquid carriers, whether sterile or not.

Preferably, the said composition optionally further comprises of one or more additional pharmaceutically acceptable carriers selected from the group consisting of a flavoring agents, sweeteners, binders, diluents, solubilizer, lubricants, suspending agents, fillers, glidants, compression aides, disintegrating agents, effervescing agents, dyestuffs, wetting agents or encapsulating materials and mixtures thereof wherein carrier is chosen from solid or liquid carriers, whether sterile or not. In powders, the carrier and compound is a finely divided solid. In tablets, said compound is mixed with a carrier having the necessary compression properties in suitable proportions and compacted in the shape and size desired. Said powders and tablets contain from 0,1 to 99% by weight of the compound. Solid carriers suitable for use in the composition of the invention include but are not limited to calcium phosphate, magnesium stearate, talc, sugars, lactose, dextrin, starch, gelatin, cellulose, methyl cellulose, sodium carboxymethyl cellulose, polyvinylpyrrolidine, low melting waxes and ion exchange resins.

Any pharmaceutically acceptable liquid carrier suitable for preparing solutions, suspensions, emulsions, syrups and elixirs can be employed in the composition of the invention. Is that case, the compound is dissolved or suspended in a pharmaceutically acceptable liquid carrier such as water, an organic solvent, or pharmaceutically acceptable oil or fat, or a mixture thereof. Said liquid composition contain additionally or not other suitable pharmaceutical additives such as solubilizers, emulsifiers, buffers, preservatives, sweeteners, flavoring agents, suspending agents, thickening agents, coloring agents, viscosity regulators, stabilizers, osmo-regulators, or the like.

Examples of liquid carriers suitable for oral and parenteral administration include water, particularly containing additives as above but not limited to cellulose derivatives, preferably sodium carboxymethyl cellulose solution, alcohols, including monohydric alcohols and polyhydric alcohols, such as glycols or their derivatives, or oils such as fractionated coconut oil, cottonseed oil and arachid oil. For parenteral administration the carrier may also be an oily ester such as ethyl oleate or isopropyl myristate.

Preferably, the said composition is a sterile solution or suspension suitable for parenteral administration, including but not limited to intramuscular, intraperitoneal, intravenous, intrathecal or subcutaneous injection or perfusion.

Preferably the pharmaceutical composition is suitable for oral administration either liquid or solid composition form, including pills, tablets, capsules, sachets, dragees, powders, granules, lozenges, powders for reconstitution, orally disintegrating tablet, films, osmotic controlled release capsule, elixir, emulsion, syrup, suspension, tincture, solutions or powder for inhalation and nebulization, or sublingual administration.

Preferably, the said composition is a formulation for transdermal, rectal, vaginal administration, including ointments, creams, lotions, liniments, gels, paste, films, suppositories, enemas and pessaries.

Preferably, the said composition is a formulation for administration through the eyes, ears and nose.

Preferably, the composition is an immediate, extended or slow- release formulation. Preferably, the therapeutically effective amount is provided in the treatment of a disease, disorders or medical condition that is selected from the group consisting of myeloid leukemia both acute and chronic, acute lymphoblastic leukemia, chronic lymphocytic leukemia, hairy cell leukemia, myeloproliferative diseases, multiple myeloma, myelodysplastic syndrome, Hodgkin's disease, non-Hodgkin's lymphoma ; adenocarcinoma, lymphoma, leukemia of the kidney, Wilm's tumor, renal cell carcinoma, renal pelvis carcinoma, nephroma, teratoma, sarcoma of the kidney, squamous cell carcinoma, transitional cell carcinoma, adenocarcinoma of bladder and urethra, sarcoma of the prostate, seminoma, embryonal carcinoma, teratocarcinoma, choriocarcinoma, sarcoma, interstitial cell carcinoma, fibroadenoma, adenomatoid tumors, lipoma of the testis, angiosarcoma, fibrosarcoma, rhabdomyosarcoma, liposarcoma, myxoma, rhabdomyoma, fibroma, lipoma and teratoma of the heart, astrocytoma, medulloblastoma, glioma, ependymoma, germinoma , glioblastoma multiform, oligodendroglioma, schwannoma, retinoblastoma, congenital tumors of the brain, neurofibroma, meningioma, sarcoma of the spinal cord, osteoma, hemangioma, granuloma, xanthoma, osteitis deformians of the skull, meningiosarcoma, gliomatosis of the meninges; squamous cell, undifferentiated small cell, undifferentiated large cell, adenocarcinoma, alveolar carcinoma, bronchial adenoma, sarcoma, lymphoma, chondromatous hanlartoma, mesothelioma of the bronchus; adenocarcinoma, lymphoma, Karposi's sarcoma, leiomyoma, lipoma, , fibroma of the small bowel, large bowel, leiomyosarcoma, lymphoma of the esophagus, carcinoma, leiomyosarcoma of the stomach, ductal adenocarcinoma, insulinoma, glucagonoma, gastrinoma, vipoma of the pancreas; hepatocellular carcinoma, cholangiocarcinoma, hepatoblastoma, angiosarcoma, hepatocellular adenoma, hemangioma of the liver; osteogenic sarcoma, fibrosarcoma, malignant fibrous histiocytoma, chondrosarcoma, Ewing's sarcoma, malignant lymphoma including reticulum cell sarcoma, malignant giant cell tumor chondroma, osteochondroma such as osteocartilaginous exostoses, benign chondroma, chondroblastoma, chondromyxofibroma, osteoid osteoma and giant cell tumors, endometrial carcinoma, cervical carcinoma, pre-tumor cervical dysplasia, ovarian carcinoma including serous cystadenocarcinoma, mucinous cystadenocarcinoma, granulosa-thecal cell tumors, Sertol/Leydig cell tumors, dysgerminoma, malignant teratoma of the ovary, intraepithelial carcinoma, adenocarcinoma, fibrosarcoma, melanoma of the vulva, clear cell carcinoma, squamous cell carcinoma, botryoid sarcoma includingembryonal rhabdomyosarcoma of the vagina, fallopian tubes carcinoma), malignant melanoma, basal cell carcinoma, moles dysplastic nevi, angioma, dermatofibroma, keloids, bone marrow transplant rejection, rheumatoid arthritis, psoriasis, type I diabetes mellitus and multiple sclerosis. Thus, the terms "cancer", "neoplasm" or "neoplastic," as provided herein, refer to a cell afflicted by any one of the above-identified conditions but are not limited thereto.

Preferably, it is for the prevention or treatment of neoplastic conditions, especially related with the modulation or regulation of serine/threonine or tyrosine kinases, preferably selected from the group of PIM, HIPK, DYRK, CLK, CDK, FLT, PKG, Haspin, MER, TAO, MNK and TRK kinases.

Serine/threonine and tyrosine kinases inhibitors disclosed in this application may also be used for treating immune disorders such as but not limited to bone marrow transplant rejection, rheumatoid arthritis, psoriasis, type I diabetes mellitus, multiple sclerosis. Inhibition of serine/threonine and tyrosine kinases can be also used for treatment of infectious diseases exemplified by but not limited to infections with herpesiviruses.

The next subject of invention is a use of the compound of Formula A as claimed in independent claim 1 for the preparation of a pharmaceutical composition comprising a therapeutically effective amount of at least one compound selected from the group consisting of compounds as described above and optionally comprising a therapeutic agent selected from a chemotherapeutic or anti-proliferative agent, an immunomodulatory or immunosuppressive agent, or an anti-inflammatory agent.

Preferably, it is used for the prevention or treatment of neoplastic or immune conditions, especially related to the modulation or regulation of serine/threonine and tyrosine kinases, preferably selected from the group of PIM, HIPK, DYRK, CLK, CDK, FLT, PKG, Haspin, MER, TAO, MNK and TRK kinases.

Preferably, for preventing or treating neoplastic conditions selected from the group consisting of myeloid leukemia both acute and chronic, acute lymphoblastic leukemia, chronic lymphocytic leukemia, hairy cell leukemia, a myeloproliferative disease, multiple myeloma, myelodysplastic syndrome, Hodgkin's disease, non-Hodgkin's lymphoma; adenocarcinoma, lymphoma, leukemia of the kidney, Wilm's tumor, renal cell carcinoma, renal pelvis carcinoma, nephroma, teratoma, sarcoma of the kidney, squamous cell carcinoma, transitional cell carcinoma, adenocarcinoma of bladder and urethra, sarcoma of the prostate, seminoma, embryonal carcinoma, teratocarcinoma, choriocarcinoma, sarcoma, interstitial cell carcinoma, fibroma, fibroadenoma, adenomatoid tumors, lipoma of the testis, angiosarcoma, fibrosarcoma, rhabdomyosarcoma, liposarcoma, myxoma, rhabdomyoma, lipoma and teratoma of the heart, astrocytoma, medulloblastoma, glioma, ependymoma, germinoma , glioblastorma multiform, oligodendroglioma, schwannoma, retinoblastoma, congenital tumors of the brain, neurofibroma, meningioma, sarcoma of the spinal cord, osteoma, hemangioma, granuloma, xanthoma, osteitis deformians of the skull, meningiosarcoma, gliomatosis of the meninges, alveolar carcinoma, bronchial adenoma, chondromatous hanlartoma, mesothelioma of the bronchus; adenocarcinoma, lymphoma, carcinoid tumors, Karposi's sarcoma, leiomyoma, lipoma, fibromaof the small bowel, adenocarcinoma, tubular adenoma, villous adenoma, hamartoma, leiomyoma of the large bowel, leiomyosarcoma, lymphoma of the esophagus, carcinoma, leiomyosarcoma of the stomach, ductal adenocarcinoma, insulinoma, glucagonoma, gastrinoma, vipoma of the pancreas; hepatocellular carcinoma, cholangiocarcinoma, hepatoblastoma, hepatocellular adenoma, hemangioma of the liver; osteogenic sarcoma, malignant fibrous histiocytoma, chondrosarcoma, Ewing's sarcoma, malignant lymphoma including reticulum cell sarcoma, malignant giant cell tumor chordoma, osteochronfroma including osteocartilaginous exostoses, benign chondroma, chondroblastoma, chondromyxofibroma, osteoid osteoma, giant cell tumors, endometrial carcinoma, cervical carcinoma, pre-tumor cervical dysplasia, ovarian carcinoma including serous cystadenocarcinoma, mucinous cystadenocarcinoma, granulosa-thecal cell tumors, Sertol/Leydig cell tumors, dysgerminoma, malignant teratoma of the ovary, intraepithelial carcinoma, melanoma of the vulva, clear cell carcinoma, botryoid sarcoma including embryonal rhabdomyosarcoma of the vagina, fallopian tubes carcinoma, breast malignant melanoma, basal cell carcinoma, squamous cell carcinoma, Karposi's sarcoma, moles dysplastic nevi, angioma, dermatofibroma, keloids, bone marrow transplant rejection, rheumatoid arthritis, psoriasis, type I diabetes mellitus and multiple sclerosis.

Preferably, serine/threonine or tyrosine kinases, preferably kinases selected from the group of PIM, HIPK, DYRK, CLK, CDK, FLT, PKG, Haspin, MER, TAO, MNK and TRK kinases is in a subject with a disease, disorders or medical condition that is selected from the group consisting of myeloid leukemia both acute and chronic, acute lymphoblastic leukemia, chronic lymphocytic leukemia, hairy cell leukemia, a myeloproliferative disease, multiple myeloma, myelodysplastic syndrome, Hodgkin's disease, non-Hodgkin's lymphoma ; adenocarcinoma, lymphoma, leukemia of the kidney, Wilm's tumor, renal cell carcinoma, renal pelvis carcinoma, nephroma, teratoma, sarcoma of the kidney, squamous cell carcinoma, transitional cell carcinoma, adenocarcinoma of bladder and urethra, sarcoma of the prostate, seminoma, embryonal carcinoma, teratocarcinoma, choriocarcinoma, sarcoma, interstitial cell carcinoma, fibroma, fibroadenoma, adenomatoid tumors, lipoma of the testis, angiosarcoma, fibrosarcoma, rhabdomyosarcoma, liposarcoma, myxoma, rhabdomyoma, lipoma and teratoma of the heart, astrocytoma, medulloblastoma, glioma, ependymoma, germinoma , glioblastorma multiform, oligodendroglioma, schwannoma, retinoblastoma, congenital tumors of the brain, neurofibroma, meningioma, sarcoma of the spinal cord, osteoma, hemangioma, granuloma, xanthoma, osteitis deformians of the skull, meningiosarcoma, gliomatosis of the meninges, alveolar carcinoma, bronchial adenoma, chondromatous hanlartoma, mesothelioma of the bronchus; adenocarcinoma, lymphoma, carcinoid tumors, Karposi's sarcoma, leiomyoma, lipoma, fibromaof the small bowel, adenocarcinoma, tubular adenoma, villous adenoma, hamartoma, leiomyoma of the large bowel, leiomyosarcoma, lymphoma of the esophagus, carcinoma, leiomyosarcoma of the stomach, ductal adenocarcinoma, insulinoma, glucagonoma, gastrinoma, vipoma of the pancreas; hepatocellular carcinoma, cholangiocarcinoma, hepatoblastoma, hepatocellular adenoma, hemangioma of the liver; osteogenic sarcoma, malignant fibrous histiocytoma, chondrosarcoma, Ewing's sarcoma, malignant lymphoma including reticulum cell sarcoma, malignant giant cell tumor chordoma, osteochronfroma including osteocartilaginous exostoses, benign chondroma, chondroblastoma, chondromyxofibroma, osteoid osteoma, giant cell tumors, endometrial carcinoma, cervical carcinoma, pre-tumor cervical dysplasia, ovarian carcinoma including serous cystadenocarcinoma, mucinous cystadenocarcinoma, granulosa-thecal cell tumors, Sertol/Leydig cell tumors, dysgerminoma, malignant teratoma of the ovary, intraepithelial carcinoma, melanoma of the vulva, clear cell carcinoma, , botryoid sarcoma such as embryonal rhabdomyosarcoma of the vagina, fallopian tubes carcinoma, breast malignant melanoma, basal cell carcinoma, squamous cell carcinoma, Karposi's sarcoma, moles dysplastic nevi, angioma, dermatofibroma, keloids, bone marrow transplant rejection, rheumatoid arthritis, psoriasis, type I diabetes mellitus and multiple sclerosis.

The next subject of invention is a process for the preparation of a compound as claimed herein characterized in that the process comprises: reacting of a corresponding, unsubstituted or substituted 2,4,5,6,7-pentabromo-benzimidazole with a suitable amine at elevated temperature, wherein the reactive substituents are optionally protected with suitable protecting groups and wherein the resulting product is subjected to purification by crystallization or chromatography according to the reaction as shown in Reaction scheme 1 below.

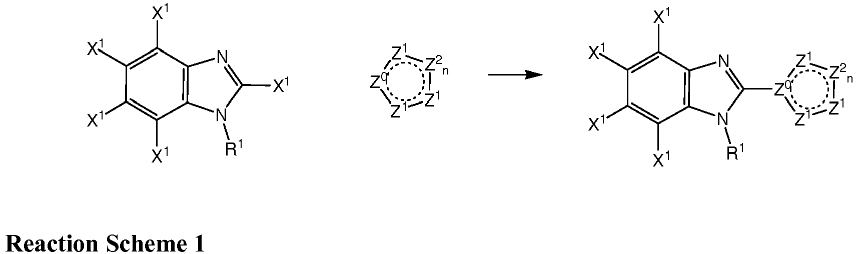

**Reaction scheme 1:** synthetic route of production of a compound by reacting an unsubstituted or substituted 2,4,5,6,7-pentahalogenated-benzimidazole with a suitable amine.

Below, there are example embodiments of the present invention defined above.

### EXAMPLES

The following specific Examples are set forth to illustrate the invention and to aid in the understanding of the invention.

The invention relates to compounds as claimed.

### General synthetic procedure A

1 equivalent of appropriate derivative of 2,4,5,6,7-pentabromobenzimidazole suspended in ethanol was heated at 120°C together with 5 equivalents of appropriate amine in a sealed tube for 5 to 20 hours. Alternatively, the reaction can be carried in butanol reflux. Product was isolated using crystallization or flash chromatography.

### General synthetic procedure B

1 equivalent of 4,5,6,7-tetrabromo-2-(piperazin-1-yl)-1*H*-benzo[*d*]imidazole or N-(3-aminopropyl)-4,5,6,7-tetrabromo-1H-benzo[*d*]imidazol-2-amine was suspended in DMF with 2 equivalents of DIPEA. Suspension was cooled to 0°C and then 2 equivalents of 1*H*-pyrazole-1-carboximidamide were added. The reaction mixture (RM) was stirred for 4 hours and then NaOH was added. Solid product was filtered and washed with distilled water.

### General synthetic procedure C

1 equivalent of 4,5,6,7-tetrabromo-2-(piperazin-1-yl)-1*H*-benzo[*d*]imidazole was suspended in EtOH with 2 equivalents of appropriate halogeno derivative and 2 eq of K₂CO₃. RM was refluxed for 2-16 hours. Product was isolated using crystallization or flash chromatography.

### General synthetic procedure D

1 equivalent of N1-(4,5,6,7-tetrabromo-1H-benzo[*d*]imidazol-2-yl)cyclohexane-1,2-diamine and 1 eq of appropriate ketone or aldehyde derivative were suspended in DCE and then 2 eq of NaBH(OAc)₃ and 1 eq ofAcOH were added. Solution was stirred at room temperature for 24 hours. Product was isolated using crystallization or flash chromatography.

### Example 1. Growth inhibition test on human chronic myelocytic leukemia K562 cells - results summarized in Table 4

Ten thousand cells of human chronic myelocytic leukemia K562 were inoculated into each well of a 96-well microplate (manufactured by Corning Corp.) using Iscove's MDM medium (culture medium) containing 10% fetal calf serum (FCS). Next day, a dimethyl sulfoxide (DMSO) solution of each test compound prepared in a concentration of 10 mmol/L was further diluted with culture medium to the desired concentrations (0.1, 0.5, 1, 2.5, 5 and 10 micromol/L), and the diluted solution was added to each well. The individual wells were further cultured in 5% carbon dioxide at 37° C for 72 hours. After completion of the culture, 10 µl MTS (3-(4,5-Dimethyl-2-thiazolyl)-5-(carboxymethoxyphenyl)-2-(4-sulfophenyl)-2H-tetrazolium, inner salt assay (CellTiter96® AQueous One Solution Cell Proliferation Assay, Promega) was added to each well, and culturing was performed in 5% carbon dioxide at 37° C for 2 hours. Using a microplate spectrophotometer (Synergy 2 multi-mode microplate reader (BioTek)), the absorbance of each well was measured at 490 nm. The value for cells not incorporated with a test compound was designated as 100%. By comparing these values with the absorbance difference obtained at the well in which each test compound was added, the cell viability (% viability) after treatment with the test compound was calculated.

### Example 2. Growth inhibition test on human prostate adenocarcinoma PC3 cells - results summarized in Table 4

Two thousand cells of human prostate adenocarcinoma PC3 were inoculated into each well of a 96-well microplate (manufactured by Corning Corp.), and using F12K (Ham's F12:RPMI 8226 1:1) medium (culture medium) containing 10% fetal calf serum (FCS). Next day, a dimethyl sulfoxide (DMSO) solution of each test compound prepared in a concentration of 10 mmol/L was further diluted with culture medium to the desired concentrations (0.1, 0.5, 1, 2.5, 5 and 10 micromol/L), and the diluted solution was added to each well. The individual wells were further cultured in 5% carbon dioxide at 37° C for 72 hours. After completion of the culture, 10 µl MTS (3-(4,5-Dimethyl-2-thiazolyl)-5-(carboxymethoxyphenyl)-2-(4-sulfophenyl)-2H-tetrazolium, inner salt assay (CellTiter96® AQueous One Solution Cell Proliferation Assay, Promega) was added to each well, and culturing was performed in 5% carbon dioxide at 37° C for 2 hours. Using a microplate spectrophotometer (Synergy 2 multi-mode microplate reader (BioTek)), the absorbance of each well was measured at 490 nm. The value for cells not incorporated with a test compound was designated as 100%. By comparing these values with the absorbance difference obtained at the well in which each test compound was added, the cell viability (% viability) after treatment with the test compound was calculated.

### Example 3. Growth inhibition test on human prostate carcinoma DU145 cells-results summarized in Table 4

Two thousand cells of human prostate carcinoma DU145 were inoculated into each well of a 96-well microplate (manufactured by Corning Corp.), and using DMEM medium (culture medium) containing 10% fetal calf serum (FCS). Next day, a dimethyl sulfoxide (DMSO) solution of each test compound prepared in a concentration of 10 mmol/L was further diluted with culture medium to the desired concentrations (0.1, 0.5, 1, 2.5, 5 and 10 micromol/L), and the diluted solution was added to each well. The individual wells were further cultured in 5% carbon dioxide at 37° C for 72 hours. After completion of the culture, 10 µl MTS (3-(4,5-Dimethyl-2-thiazolyl)-5-(carboxymethoxyphenyl)-2-(4-sulfophenyl)-2H-tetrazolium, innersalt assay (CellTiter96® AQueous One Solution Cell Proliferation Assay, Promega) was added to each well, and culturing was performed in 5% carbon dioxide at 37° C for 2 hours. Using a microplate spectrophotometer (Synergy 2 multi-mode microplate reader (BioTek)), the absorbance of each well was measured at 490 nm. The value for cells not incorporated with a test compound was designated as 100%. By comparing these values with the absorbance difference obtained at the well in which each test compound was added, the cell viability (% viability) after treatment with the test compound was calculated.

### Example 4: Growth inhibition test on human breast adenocarcinoma MCF7 cells - results summarized in Table 4

Two thousand cells of human breast adenocarcinoma MCF7 were inoculated into each well of a 96-well microplate (manufactured by Corning Corp.), and using DMEM medium (culture medium) containing 10% fetal calf serum (FCS). Next day, a dimethyl sulfoxide (DMSO) solution of each test compound prepared in a concentration of 10 mmol/L was further diluted with culture medium to the desired concentrations (0.1, 0.5, 1, 2.5, 5 and 10 micromol/L), and the diluted solution was added to each well. The individual wells were further cultured in 5% carbon dioxide at 37°C for 72 hours. After completion of the culture, 10 µl MTS (3-(4,5-Dimethyl-2-thiazolyl)-5-(carboxymethoxyphenyl)-2-(4-sulfophenyl)-2H-tetrazolium, inner salt assay (CellTiter96® AQueous One Solution Cell Proliferation Assay, Promega) was added to each well, and culturing was performed in 5% carbon dioxide at 37°C for 2 hours. Using a microplate spectrophotometer (Synergy 2 multi-mode microplate reader (BioTek)), the absorbance of each well was measured at 490 nm. The value for cells not incorporated with a test compound was designated as 100%. By comparing these values with the absorbance difference obtained at the well in which each test compound was added, the cell viability (% viability) after treatment with the test compound was calculated.

### Example 5: Growth inhibition test on human colorectal adenocarcinoma SW480 cells - results summarized in Table 4

Two thousand cells of human colorectal adenocarcinoma SW480 were inoculated into each well of a 96-well microplate (manufactured by Corning Corp.), and using DMEM/ Ham's F121:1 medium (culture medium) containing 5% fetal calf serum (FCS). Next day, a dimethyl sulfoxide (DMSO) solution of each test compound prepared in a concentration of 10 mmol/L was further diluted with culture medium to the desired concentrations (0.1, 0.5, 1, 2.5, 5 and 10 micromol/L), and the diluted solution was added to each well. The individual wells were further cultured in 5% carbon dioxide at 37°C for 72 hours. After completion of the culture, 10 µl MTS (3-(4,5-Dimethyl-2-thiazolyl)-5-(carboxymethoxyphenyl)-2-(4-sulfophenyl)-2H-tetrazolium, inner salt assay (CellTiter96® AQueous One Solution Cell Proliferation Assay, Promega) was added to each well, and culturing was performed in 5% carbon dioxide at 37°C for 2 hours. Using a microplate spectrophotometer (Synergy 2 multi-mode microplate reader (BioTek)), the absorbance of each well was measured at 490 nm. The value for cells not incorporated with a test compound was designated as 100%. By comparing these values with the absorbance difference obtained at the well in which each test compound was added, the cell viability (% viability) after treatment with the test compound was calculated.

### Example 6: Growth inhibition test on human myelomonocytic, biphenotypic leukemia MV4-11 cells - results summarized in Table 4

Ten thousand cells of human myelomonocytic, biphenotypic leukemia MV4-11 were inoculated into each well of a 96-well microplate (manufactured by Corning Corp.)using Iscove's MDM medium (culture medium) containing 10% fetal calf serum (FCS). Next day, a dimethyl sulfoxide (DMSO) solution of each test compound prepared in a concentration of 10 mmol/L was further diluted with culture medium to the desired concentrations (0.1, 0.5, 1, 2.5, 5 and 10 micromol/L), and the diluted solution was added to each well. The individual wells were further cultured in 5% carbon dioxide at 37°C for 72 hours. After completion of the culture, 10 µl MTS (3-(4,5-Dimethyl-2-thiazolyl)-5-(carboxymethoxyphenyl)-2-(4-sulfophenyl)-2H-tetrazolium, inner salt assay (CellTiter96® AQueous One Solution Cell Proliferation Assay, Promega) was added to each well, and culturing was performed in 5% carbon dioxide at 37°C for 2 hours. Using a microplate spectrophotometer (Synergy 2 multi-mode microplate reader (BioTek)), the absorbance of each well was measured at 490 nm. The value for cells not incorporated with a test compound was designated as 100%. By comparing these values with the absorbance difference obtained at the well in which each test compound was added, the cell viability (% viability) after treatment with the test compound was calculated.

### Example 7: Growth inhibition test on human acute lymphocytic leukemia E6.1 Jurkat cells - results summarized in Table 4

Twenty thousand cells of human acute lumphocytic leukemia E6.1 Jurkat were inoculated into each well of a 96-well microplate (manufactured by Corning Corp.) using RPMI 8226 medium (culture medium) containing 10% fetal calf serum (FCS). Next day, a dimethyl sulfoxide (DMSO) solution of each test compound prepared in a concentration of 10 mmol/L was further diluted with culture medium to the desired concentrations (0.1, 0.5, 1, 2.5, 5 and 10 micromol/L), and the diluted solution was added to each well. The individual wells were further cultured in 5% carbon dioxide at 37°C for 72 hours. After completion of the culture, 10 µl MTS (3-(4,5-Dimethyl-2-thiazolyl)-5-(carboxymethoxyphenyl)-2-(4-sulfophenyl)-2H-tetrazolium, inner salt assay (CellTiter96® AQueous One Solution Cell Proliferation Assay, Promega) was added to each well, and culturing was performed in 5% carbon dioxide at 37°C for 2 hours. Using a microplate spectrophotometer (Synergy 2 multi-mode microplate reader (BioTek)), the absorbance of each well was measured at 490 nm. The value for cells not incorporated with a test compound was designated as 100%. By comparing these values with the absorbance difference obtained at the well in which each test compound was added, the cell viability (% viability) after treatment with the test compound was calculated.

### Example 8: Growth inhibition test on human hepatoma HepG2 cells - results summarized in Table 4

Two thousand cells of human hepatoma HepG2 were inoculated into each well of a 96-well microplate (manufactured by Corning Corp.) using DMEM medium (culture medium) containing 10% fetal calf serum (FCS). Next day, a dimethyl sulfoxide (DMSO) solution of each test compound prepared in a concentration of 10 mmol/L was further diluted with culture medium to the desired concentrations (0.1, 0.5, 1, 2.5, 5 and 10 micromol/L), and the diluted solution was added to each well. The individual wells were further cultured in 5% carbon dioxide at 37°C for 72 hours. After completion of the culture, 10 µl MTS (3-(4,5-Dimethyl-2-thiazolyl)-5-(carboxymethoxyphenyl)-2-(4-sulfophenyl)-2H-tetrazolium, inner salt assay (CellTiter96® AQueous One Solution Cell Proliferation Assay, Promega) was added to each well, and culturing was performed in 5% carbon dioxide at 37°C for 2 hours. Using a microplate spectrophotometer (Synergy 2 multi-mode microplate reader (BioTek)), the absorbance of each well was measured at 490 nm. The value for cells not incorporated with a test compound was designated as 100%. By comparing these values with the absorbance difference obtained at the well in which each test compound was added, the cell viability (% viability) after treatment with the test compound was calculated.

### Example 9: Growth inhibition test on human erytroblast leukemia HEL-92.1.7 cells - results summarized in Table 4

Ten thousand cells of human erytroblast leukemia HEL-92.1.7 were inoculated into each well of a 96-well microplate (manufactured by Corning Corp.) using RPMI 1640 medium (culture medium) containing 10% fetal calf serum (FCS). Next day, a dimethyl sulfoxide (DMSO) solution of each test compound prepared in a concentration of 10 mmol/L was further diluted with culture medium to the desired concentrations (0.1, 0.5, 1, 2.5, 5 and 10 micromol/L), and the diluted solution was added to each well. The individual wells were further cultured in 5% carbon dioxide at 37°C for 72 hours. After completion of the culture, 10 µl MTS (3-(4,5-Dimethyl-2-thiazolyl)-5-(carboxymethoxyphenyl)-2-(4-sulfophenyl)-2H-tetrazolium, inner salt assay (CellTiter96® AQueous One Solution Cell Proliferation Assay, Promega) was added to each well, and culturing was performed in 5% carbon dioxide at 37°C for 2 hours. Using a microplate spectrophotometer (Synergy 2 multi-mode microplate reader (BioTek)), the absorbance of each well was measured at 490 nm. The value for cells not incorporated with a test compound was designated as 100%. By comparing these values with the absorbance difference obtained at the well in which each test compound was added, the cell viability (% viability) after treatment with the test compound was calculated.

### Example 10: Luminometric kinase assay for PIM1 kinase- results summarized in Table 4

Kinase assay was performed using luminescent Kinase-Glo® (Promega) system. The Kinase-Glo® Luminescent Kinase Assay Platform provides a homogeneous, high-throughput screening method for measuring kinase activity by quantifying the amount of ATP remaining in solution following a kinase reaction. The luminescent signal is correlated with the amount of ATP present and is inversely correlated with the kinase activity. Appropriate amounts of both kinase and substrate were mixed in 96-well, white wall plate in the reaction buffer (8 mM MOPS/NaOH, pH7.0, 0,2mM EDTA, 2mM MgCl₂). Tested compounds were diluted in DMSO and serial dilutions starting with 1 µM were added to the palates. Reaction was initiated by adding 1 µM of ATP solution and conducted for 25 min. at RT. Luminescent signal was detected by adding Kinase-Glo® reagent and measured using a microplate spectrophotometer (Synergy 2 multi-mode microplate reader (BioTek)). IC50 values were obtained by fitting sigmoidal dose-response curve (variable slope) using GraphPad Prism software.

Reaction conditions are presented in Table 3.

**Table 3. Reaction conditions for luminometric kinase assay.**

| **Kinase** | **Kinase concentration** | **Peptide substrate sequence** | **Peptide concentration** | **ATP concentration** |
|---|---|---|---|---|
| **PIM-1** | 6ng | KKRNRTLTK | 100 µM | 1 µM |

### Table 4. In vitro activity of compounds of Formula A.

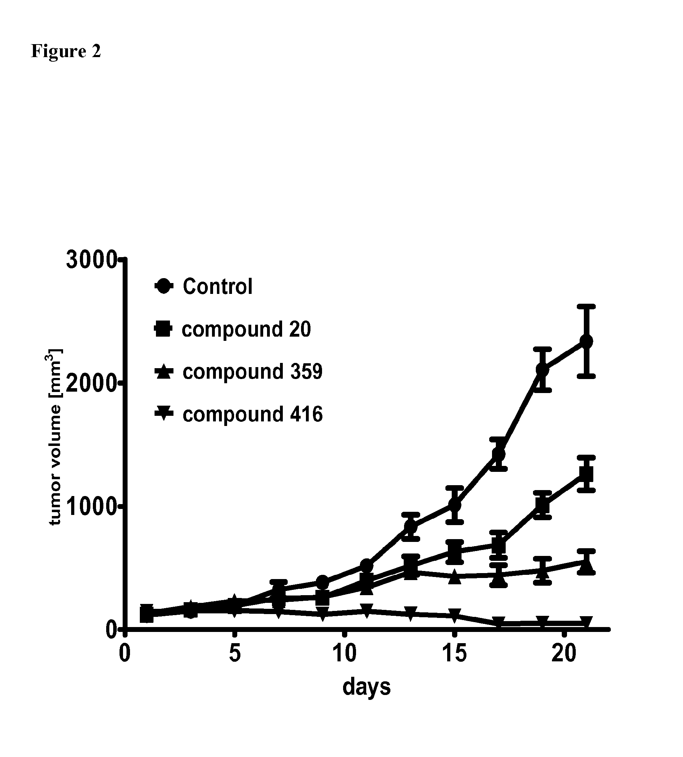

As reference compounds previously described halogenated derivatives of benzotriazole, benzimidazole and benzopyrazole were used (Pagano et al., Biochem. J. 2008 415, 353-365), Structures of used reference compounds DMAT, TBB, TBI, K66 and K64 are depicted on Figure 2 of the above publication.
- DMAT:: 2-dimethylamino-4,5,6,7-tetrabromo-1*H*-benzimidazole
- TBB:: 4,5,6,7-tetrabromo-1*H*benzotriazole
- TBI- or TBBz:: 4,5,6,7-tetrabromo-1*H*-benzimidazole
- K66:: 1-carboxymethyl-2-dimethylamino-4,5,6,7-tetrabromo-benzimidazole
- K64:: 3,4,5,6,7-pentabromo-1*H*-indazole

| **Compound number** | **PIM-1 IC50 [nM]** | **MV-4-11 ED50 [µM]** | **Jurkat ED50 [µM]** | **K562 ED50 [µM]** | **HEL92 ED50 [µM]** | **MCF-7 ED50 [µM]** | **SW480 ED50 [µM]** | **PC-3 ED50 [µM]** | **HepG 2 ED50 [µM]** |
|---|---|---|---|---|---|---|---|---|---|
| **DMAT** | 31,02 | 3,15 | 8,18 | >10 | | >10 | >10 | >10 | >10 |
| **TBB** | >1000 | >10 | >10 | >10 | | | >10 | >10 | >10 |
| **TBI** | 101,85 | >10 | >10 | >10 | | | 7,21 | >10 | >10 |
| **K66** | >1000 | >10 | >10 | >10 | | | >10 | >10 | >10 |
| **K64** | >1000 | >10 | >10 | >10 | | | 9,13 | >10 | >10 |
| **18** | 13,32 | 7,68 | | | | | | | |
| **19** | 29,4 | 6,83 | 4,63 | | | | | | |
| **20** | 16,95 | 0,48 | 2,19 | 6,46 | 6,04 | 2,47 | 3,45 | 5,14 | 2,86 |
| **21** | 41,9 | 3,78 | 2,63 | 7,99 | | | 5,98 | | 6,32 |
| **75** | 278,8 | 3,33 | 7,79 | | | | | | 5,85 |
| **76** | 117,2 | 5,27 | 3,06 | | | | | | 4,54 |
| **256** | | 2,6 | 4,86 | | | 5,11 | | 5,18 | 3,39 |
| **260** | 29,92 | 1,45 | 5,35 | | | 5,65 | | 5,59 | 7,04 |
| **262** | 61,36 | | | | | | | | |
| **266** | 293,9 | 2,55 | | | | | | | 2,48 |
| **343** | 36,79 | | | | | | | | |
| **350** | 32,73 | 5,52 | | | | 6,55 | | 8,48 | 5,55 |
| **353** | 70,80 | 7,53 | | | ∼10 | | | | 8,01 |
| **359** | 6,84 | 0,6 | | 3,05 | 3,85 | 1,6 | 1,6 | 2,95 | 1,85 |
| **364** | 10,25 | 1,97 | | 5,21 | 5,57 | 1,6 | 1,27 | 4,58 | 2,33 |
| **367** | 39,48 | 3,77 | | | | | | | |
| **374** | 107,03 | 1,3 | | 2,94 | 3 | 1,5 | 1,44 | 2,5 | 1,33 |
| **376** | | 5,17 | | | 7,93 | | | 6,61 | 9,4 |
| **377** | | 5,92 | | | 9,73 | | | 5,99 | 9,72 |
| **378** | 39,13 | 6,15 | | | 9,04 | 6,19 | 5 | 6,66 | 4,2 |
| **383** | 94,40 | 3,43 | | | | 8,32 | 3,72 | 8,3 | 7,12 |
| **385** | 45,29 | 4,57 | | | 2,21 | 5,8 | | 6,88 | 3,76 |
| **386** | 57,96 | 3,31 | | 2,86 | 6,12 | 2,47 | 1,77 | 2,77 | |
| **387** | | 2,13 | | | | 9,25 | 3,27 | 5,49 | |
| **392** | 6,72 | 7,55 | | | | | 1,85 | 4,48 | |
| **414** | | 3,03 | | | 7,1 | 8,23 | 4,32 | 8,94 | |
| **416** | 6,55 | 0,55 | | 3,65 | 2,72 | 1,26 | 1,25 | 2,1 | 1,31 |
| **417** | 9,36 | 0,56 | | 4,96 | 3,28 | 2,54 | 2,56 | 2,57 | 1,85 |
| **441** | 65 | 0,7 | | 1,65 | 0,6 | 1,3 | 0,7 | 1,6 | 0,7 |
| **450** | 125 | 0,6 | | 2,9 | 0,8 | 2 | 1 | 3,6 | 1,5 |

### Example 11: Kinase panel results

### Kinase panel profiling was performed at Millipore

(http://www.millipore.com/drugdiscovery/dd3/KinaseProfiler) using the Millipore's KinaseProfilerTM service, a high-throughput method for screening small molecule compounds against large numbers of different wild type and mutant kinases. This kinase panel is based on radiometric assay that quantitatively measures the ability of a compound to prevent phosphorylation of a peptide substrate. The radiometric based filtration binding assay is considered to be the "gold standard" to which other non-radiometric methods are compared. In this assay type, the kinase reaction is performed in the presence of radioactive ATP isotope followed by binding of the final radioisotope labeled products to filters. After the reaction was performed, unreacted phosphate is washed away and the levels of phosphorylated radioactive substrate are measured. Selectivity of kinase inhibition is one of the key parameters in therapeutic use of this class of compounds. Off-target inhibition could be one of the major safety and toxicity issues in drug development and use of the compounds for treatment of various disease conditions, but also can be one of the reasons of potency and anticancer effect displayed by the compound. Several examples of kinase inhibitors are described in the literature for which after a more detailed and broad analysis is was shown, that the activity is not related to primary target.

Therefore representative compounds disclosed in the application were tested on a panel kinases to determine specificity in PIM kinase inhibition. Table 5 provides selectivity data for representative compound 4,5,6,7-tetrabromo-1-ethyl-2-(piperazin-1-yl)-1H-benzo[d]imidazole (compound 416). As indicated in the Table 5 when tested at 1 µM concentration 4,5,6,7-tetrabromo-1-ethyl-2-(piperazin-1-yl)-1H-benzo[d]imidazole was revealed to be not only a potent inhibitor of PIM kinases, but also exerts activity on a set of other kinases.

**Table 5. KinomeScan Max kinase binding inhibition panel results for 4,5,6,7-tetrabromo-1-ethyl-2-(piperazin-1-yl)-1H-benzo[d]imidazole tested at 1 µM in duplicate. Values represent % of remaining kinase activity**

| Kinase | % remaining activity | Kinase | % remaining activity | Kinase | % remaining activity | Kinase | % remaining activity |
|---|---|---|---|---|---|---|---|
| HIPK3(h) | **-1** | **CK1δ(h)** | **62** | **JNK3(h)** | **87** | **Abl (Q252H) (h)** | **98** |
| Flt3(h) | **0** | **Hck(h) activated** | **63** | **MINK(h)** | **87** | **LIMK1(h)** | **98** |
| CDK5/p25(h) | **1** | **Lyn(h)** | **65** | **MLK1(h)** | **87** | **PDGFR**α**(h)** | **98** |
| Flt3(DS35Y)(h) | **1** | **Src(1-530)(h)** | **65** | **MSSK1(h)** | **87** | **PKCβII(h)** | **98** |
| HIPK2(h) | **1** | **GSK3β(h)** | **66** | **TGFBR1(h)** | **87** | **EphA4(h)** | **99** |
| PIM-1(h) | **1** | **STK33(h)** | **66** | **MLCK(h)** | **88** | **EphA5(h)** | **99** |
| CDK5/p35(h) | **2** | **AMPKα2(h)** | **67** | **PhK**γ**2(h)** | **88** | **FGFR1(h)** | **99** |
| CLK2(h) | **2** | **CaMKIIβ(h)** | **67** | **ULK2(h)** | **88** | **PTK5(h)** | **99** |
| Haspin(h) | **2** | **Fgr(h)** | **67** | **CaMKI(h)** | **89** | **SAPK2a(h)** | **99** |
| PKG1β(h) | **5** | **PDGFRα(V561D)(h)** | **68** | **CK1**γ**3(h)** | **89** | **Txk(h)** | **99** |
| CDK2/cyclinE(h) | **6** | **SAPK3(h)** | **68** | **EGFR(h)** | **89** | **ASK1(h)** | **100** |
| CK1(y) | **6** | **FGFR1(V561M)(h)** | **69** | **ErbB4(h)** | **89** | **DAPK1(h)** | **100** |
| cKit(D816H)(h) | **6** | **Lck(h) activated** | **69** | **PKC**δ**(h)** | **89** | **EphB4(h)** | **100** |
| TAO1(h) | **6** | **Met(M1268T)(h)** | **70** | **Rsk4(h)** | **89** | **Fer(h)** | **100** |
| CDK1/cyclinB(h) | **7** | **Met(Y1248H)(h)** | **70** | **Abl(m)** | **90** | **JNK2**α**2(h)** | **100** |
| CDK2/cyclinA(h) | **7** | **PKA(h)** | **70** | **PKB**β**(h)** | **90** | **MKK4(m)** | **100** |
| PKG1α(h) | **7** | **MST1(h)** | **71** | **ROCK-I(h)** | **90** | **PAK3(h)** | **100** |
| HEPK1(H) | **8** | **ROCK-Π(r)** | **71** | **SGK(h)** | **90** | **DCAMKL2(h)** | **101** |
| PIM-2(h) | **10** | **Rsk3(h)** | **71** | **Snk(h)** | **90** | **FGFR2(N549H)(h)** | **101** |
| EGFR(T790M,L858R)(h) | **11** | **Fms(h)** | **72** | **BrSK2(h)** | **91** | **MAPKAP-K2(h)** | **101** |
| Mer(h) | **14** | **Met(D1246H)(h)** | **72** | **CDK6/cyclinD3(h)** | **91** | **Plk3(h)** | **101** |
| TAO3(h) | **14** | **CK2α2(h)** | **73** | **CSK(h)** | **91** | **Tie2(R849W)(h)** | **101** |
| CDK3/cyclinE(h) | **16** | **Fyn(h)** | **73** | **EphA7(h)** | **91** | **BTK(R28H)(h)** | **102** |
| MSK2(h) | **16** | **Lyn(m)** | **73** | **FGFR2(h)** | **91** | **EphA8(h)** | **102** |
| Flt4(h) | **17** | **MEK1(h)** | **73** | **GRK6(h)** | **91** | **PDGFRβ(h)** | **102** |
| Mnk2(h) | **17** | **Met(Y1248C)(h)** | **73** | **IRR(h)** | **91** | **PKCβI(h)** | **102** |
| PDGFRα(D842V)(h) | **17** | **NEK11(h)** | **73** | **MARK1(h)** | **91** | **SRPK2(h)** | **102** |
| Ret(VS04M)(h) | **20** | **JAK3(h)** | **74** | **NEK2(h)** | **91** | **WNK2(h)** | **102** |
| EGFR(L858R)(h) | **22** | **Met(D1246N)(h)** | **74** | **PRAK(h)** | **91** | **BrSK1(h)** | **103** |
| CDK7/cyclinH/MAT1(h) | **25** | **PrKX(h)** | **74** | **SAPK2b(h)** | **91** | **mTOR/FKBP12(h)** | **103** |
| PKCθ(H) | **26** | **EphA1(h)** | **75** | **Tie2(V897S)(h)** | **91** | **MuSK(h)** | **103** |
| Ret (V804L)(h) | **26** | **GCK(h)** | **75** | **Abl(T315I)(h)** | **92** | **VRK2(h)** | **103** |
| CDK9/cyclin T1(h) | **27** | **IGF-1R(h)** | **75** | **Abl(Y253F)(h)** | **92** | **IR(h)** | **104** |
| EGFR(L861Q)(h) | **27** | **BRK(h)** | **76** | **DAPK2(h)** | **92** | **PKCε(h)** | **104** |
| Ret(h) | **27** | **CHK2(h)** | **76** | **Fms(Y969C)(h)** | **92** | 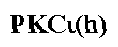 | **104** |
| CaMKIIδ(h) | **28** | **CHK2(R145W)(h)** | **76** | **PKCη(h)** | **92** | **Ros(h)** | **104** |
| EGFR(T790M)(h) | **28** | **IKKβ(h)** | **78** | **SGK3(h)** | **92** | **TBK1(h)** | **104** |
| KDR(H) | **28** | **cSRC(h)** | **79** | **Tie2(h)** | **92** | **TLK2(h)** | **104** |
| PASK(h) | **28** | **Hck(h)** | **79** | **ARK5(h)** | **93** | **DDR2(h)** | **105** |
| PKCµ(h) | **30** | **PAK4(h)** | **79** | **GRK7(h)** | **93** | **FGFR3(h)** | **105** |
| IRAK1(h) | **33** | **RIPK2(h)** | **79** | **IR(h), activated** | **93** | **Itk(h)** | **105** |
| PIM-3(h) | **34** | **Rskl(r)** | **79** | **MST2(h)** | **93** | **Rse(h)** | **105** |
| cKit(V560G)(h) | **35** | **SGK2(h)** | **79** | **MST3(h)** | **93** | **ALK(h)** | **106** |
| TRKA(h) | **35** | **Aurora-B(h)** | **80** | **PAK5(h)** | **93** | **EphA2(h)** | **106** |
| Axl(h) | **36** | **CHK2(I157T)(h)** | **80** | **PKCγ(h)** | **93** | **EphB2(h)** | **106** |
| cKit(V654A)(h) | **36** | **MAPK2(h)** | **81** | **Bmx(h)** | **94** | **MAPKAP-K3(h)** | **106** |
| TRKB(h) | **37** | **Met(h)** | **81** | **CaMKIδ(h)** | **94** | **SRPK1(h)** | **106** |
| PYBγ(h) | **39** | **Met(Y1248D)(h)** | **81** | **EphA3(h)** | **94** | **Abl(h)** | **107** |
| Flt1(h) | **40** | **ROCK-II(h)** | **81** | **FAK(h)** | **94** | **Abl(H396P) (h)** | **107** |
| Src(T341M)(h) | **40** | **Rsk2(h)** | **81** | **FGFR4(h)** | **94** | **BTK(h)** | **107** |
| CaMKIIγ(h) | **41** | **AMPKα1(h)** | **82** | **MAPK1(h)** | **94** | **eEF-2K(h)** | **107** |
| Lck(h) | **43** | **CLK3(h)** | **82** | **WNK3(h)** | **94** | **PKCζ(h)** | **107** |
| MKK6(h) | **43** | **DRAK1(h)** | **82** | **Abl(M351T)(h)** | **95** | **Tec(h) activated** | **107** |
| DYRK2(h) | **45** | **MAPK2(m)** | **82** | **MKK7β(h)** | **95** | **Ron(h)** | **108** |
| Yes(h) | **48** | **TAO2(h)** | **82** | **mTOR(h)** | **95** | **ZAP-70(h)** | **108** |
| LOK(h) | **50** | **CK1γ1(h)** | **83** | **PAK2(h)** | **95** | **PDK1(h)** | **109** |
| SAPK4(h) | **51** | **SIK(h)** | **84** | **PKCα(h)** | **95** | **ALK4(h)** | **112** |
| Fes(h) | **52** | **ULK3(h)** | **84** | **Syk(h)** | **95** | **CHK1(h)** | **113** |
| IRAK4(h) | **52** | **CaMKIV(h)** | **85** | **TSSK2(h)** | **95** | **MRCKβ(h)** | **113** |
| TAK1(H) | **52** | **CK1γ2(h)** | **85** | **ZIPK(h)** | **95** | **NEK7(h)** | **115** |
| MSK1(h) | **54** | **c-RAF(h)** | **85** | **Arg(m)** | **96** | **Plk1(h)** | **117** |
| IKKα(h) | **56** | **NEK3(h)** | **85** | **GRK5(h)** | **96** | **Arg(h)** | **119** |
| p70S6K(h) | **56** | **NLK(h)** | **85** | **MRCKα(h)** | **96** | **cKit(h)** | **121** |
| PRK2(h) | **56** | **PAK6(h)** | **85** | **TSSK1(h)** | **96** | **EphB3(h)** | **121** |
| Rsk1(h) | **56** | **PAR-1Bα(h)** | **85** | **ACK1(h)** | **97** | **JAK2(h)** | **121** |
| PKD2(h) | **58** | **LKB1(h)** | **86** | **Blk(m)** | **97** | **Aurora-A(h)** | **125** |
| CK2(h) | **59** | **MELK(h)** | **86** | **DMPK(H)** | **97** | | |
| PKBα(h) | **59** | **SAPK2a(T106M)(h)** | **86** | **JNK1α1(h)** | **97** | | |
| GSK3α(h) | **60** | **EphB1(h)** | **87** | **NEK6(h)** | **97** | | |
| cKit(D816V)(h) | **61** | **IGF-1R(h), activated** | **87** | **Pyk2(h)** | **97** | | |

### Example 12: In vitro inhibition of protein phosphorylation

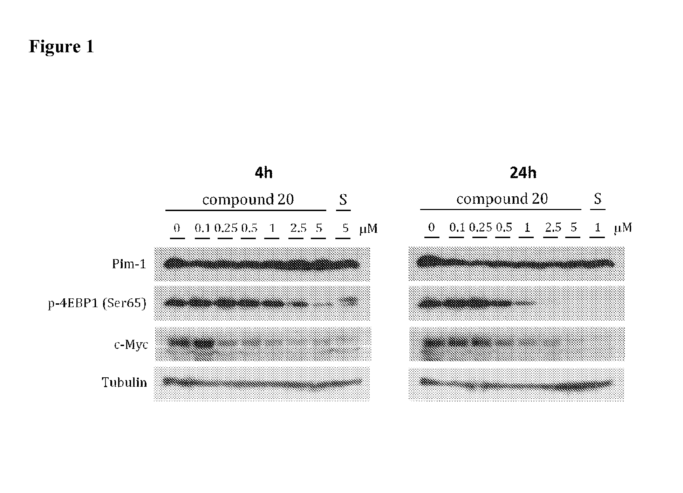

The inventors have investigated the efficacy of PIM-1 kinase inhibition by compound 20 on MV4-11 cells 4h and 24h after the treatment. The efficacy of PIM-1 inhibition was evaluated basing on the changes in the expression and phosphorylation levels of its downstream target proteins, using Western blot (Figure 1). Sunitinib was used as a positive control. After densitometric quantification of the obtained results, cellular IC50 was assessed (Table 6). The analysis revealed dose dependent inhibition of c-MYC after 4h and 24h being most efficient after 4h. Similarly, another biomarker, p-4EBP1(Ser65), showed dose- and time-dependent inhibition. PIM-1 and tubulin protein levels were assessed as controls.

**Table 6. Comparison of calculated cellular IC50 for biomarker inhibition in MV411 cells after 4 and 24h treatment with compound 20. Values are given in µM concentration.**

| | compound 20 | |
|---|---|---|
| | 4h | 24h |
| c-MYC | 0,89 | 2,03 |
| p-4EBP1 (Ser65) | 3,13 | 1,12 |

### Example 13: In vivo anticancer activity

The anticancer activity of compounds 20, 359 and 416 was assessed on tumors derived from MV4-11 cells xenografted in nude mice. Mice were inoculated with 5*10⁶ cells and the tumor volume was measured until it reached 100 mm³. Then, compounds were administered per os (PO), every day (QD) for 21 days, at the doses as indicated in the Table 7. Throughout the whole study tumor volume has been measured (Figure 2) and tumor growth inhibition (TGI) was calculated. After 21 days of treatment, compounds 20 (dose 150 mg/kg, administration QD), 359 and 416 showed the most pronounced tumor growth inhibition (Table 7).

**Table 7. Comparison of xenograft results after treatment with compound 20, -359, and -416 compounds. TGI - tumor growth inhibition, QD - once a day.**

| Compound | Dose | TGI (%) |
|---|---|---|
| Control | - | 0 |
| compound 20 | 150 mg/kg, QD | -57,8 |
| compound 359 | 150 mg/kg, QD | -84,7 |
| compound 416 | 150 mg/kg, QD | -102,8 |

### Example 14: Solubility comparison of the compounds in pH 7.4 - results summarized in Table 8

Compound stock solutions were prepared in DMSO to final concentrations of 1000 mM. For each compound studied, 12 solutions were prepared to cover concentrations from 0.001 to 1 mM. As a solvent 0.2 mM phosphate buffer (pH 7.4) was used. The final concentration of DMSO in the solutions was 1% (v/v). The solutions were left mixing for 24 hours at 37°C, 350 rpm and following incubation centrifuged for 5 minutes at 14500 rpm. The samples were then analyzed by RP HPLC using a C18 column and 0.2% solution of formic acid in water/acetonitrile mobile phase. For low concentration solutions the HPLC signal dependence on compound concentration is linear and reaches plateau for higher concentrations. The solubility was determined as the point as the point at which the concentration curve reaches plateau. Examples of the results for chosen compounds are shown in Table 8. In general, tetrahalogenated benzimidazoles are poorly soluble compounds, what renders their chances for successful therapeutic administration in commonly used routes of administration and standard conditions. In contrast to previously published tetrabromobenzimidazoles exemplified by DMAT, certain compounds provided in the application show a markedly increase in water solubility. As solubility is one of the key parameters that influences compound pharmacokinetics, permeability and therefore also activity *in vitro* and *in vivo*, improved solubility of the selected compounds is a surprising feature that can lead to improved efficacy in treating PIM kinase associated disease conditions.

**Table 8. Solubility of the compounds in pH 7.4.**

| **Compound No.** | **Solubility [mM] in phosphate buffer pH 7.4** |
|---|---|
| **DMAT** | <0,01 |
| **Compound 20** | 0,081 |

## Claims

1. A compound of formula (A): 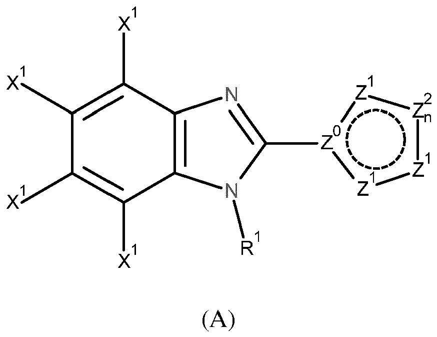 wherein:
X¹ is independently selected at each occurrence from F, Cl, or Br;
Z⁰ is selected from C, CH, and N;
Z¹ is independently selected at each occurrence from CR², CHR³, N, NR⁴, and O;
Z² is independently selected at each occurrence from CR², CHR³, N, NR⁴, O, and S;
n is 1, 2, 3, or 4 to form a 5-, 6-, 7-, or 8-membered carbocycle or heterocycle, which is saturated or unsaturated but not aromatic;
Z¹ and Z² are optionally taken together independently to form a second and optionally a third fused ring to form at least one 4-, 5-, 6-, or 7-membered carbocycle or heterocycle, which is saturated or unsaturated or aromatic and which is optionally substituted with one or more substituents selected from H, halogen, amino, hydroxyl, aminoalkyl, alkoxy, carboxylic acid, carboxyester, carboxamide, carbamate, sulfonic acid, sulfonamide and trifluoromethyl, wherein said 4- to 7- membered heterocyclic groups contain one or more heteroatom(s) selected from N, O, and S;
R¹ is selected from the group consisting of H, methyl, carboxyester, carboxamide, sulfonamide, -(C₂₋₆alkyl)R_{A}, and 5- to 8-membered carbocyclic and heterocyclic groups, which are saturated, unsaturated, or aromatic, which are optionally substituted with one or more substitutents selected from H, halogen, amino, hydroxyl, aminoalkyl, alkoxy, carboxylic acid, carboxyester, carboxamide, carbamate, sulfonic acid, sulfonamide and trifluoromethyl, wherein said 5- to 8- membered heterocyclic groups contain one or more heteroatom(s) selected from N, O, and S, with point of attachment being carbon, wherein said -(C₂₋₆alkyl)R_{A} is optionally branched and further substituted with one or more substituent(s) selected from oxo, hydroxyl, and amino;
R² is independently selected at each occurrence from the group consisting of H, halogen, amino, hydroxyl, oxo, aminoalkyl, alkoxy, carbonyl, carboxylic acid, carboxyester, carboxamide, carbamate, sulfonic acid, sulfonamide,-Y¹(C₁₋₆alkyl)R_{A}, and Y¹R_{B}, wherein said -Y¹(C₁₋₆alkyl)R_{A} is optionally branched and further substituted with one or more substituent(s) selected from oxo, hydroxyl, and amino;
R³ is independently selected at each occurrence from the group consisting of H, amino, hydroxyl, aminoalkyl, alkoxy, carboxylic acid, carboxyester, carboxamide, carbamate, sulfonic acid, sulfonamide, trifluoromethyl, -Y¹(C₁₋₆alkyl)R_{A}, and Y¹R_{B}, wherein said - Y¹(C₁₋₆alkyl)R_{A} is optionally branched and further substituted with one or more substituent(s) selected from oxo, hydroxyl, and amino;
R⁴ is independently selected at each occurrence from the group consisting of H, carboxyester, carboxamide, carbamate, sulfonamide, amidine, -Y¹(C₁₋₆alkyl)R_{A}, and R_{B} with the proviso that the point of attachment on R_{B} is carbon, wherein said -Y¹(C₁₋₆alkyl)R_{A} is optionally branched and further substituted with one or more substituent(s) selected from oxo, hydroxyl, nitrile and amino;
R_{A} is independently selected at each occurrence from the group consisting of H, amino, hydroxyl, aminoalkyl, alkoxy, carboxylic acid, carboxyester, carboxamide, carbamate, sulfonic acid, sulfonamide, trifluoromethyl, 5- to 9-membered mono- or bicyclic carbocyclic and heterocyclic groups, which are saturated, unsaturated, or aromatic, which are optionally substituted with one or more substitutents selected from H, halogen, amino, hydroxyl, alkyl, aminoalkyl, alkoxy, carboxylic acid, carboxyester, carboxamide, carbamate, sulfonic acid, sulfonamide, trifluoromethyl, substituted or unsubstituted aryl and heteroaryl, wherein said 5-to 9- membered heterocyclic groups contain one or more heteroatom(s) selected from N, O, and S, with point of attachment being carbon or nitrogen;
R_{B} is independently selected at each occurrence from the group consisting of 5- to 8-membered carbocyclic and heterocyclic groups, which are saturated, unsaturated, or aromatic, which are optionally substituted with one or more substitutents selected from H, halogen, amino, hydroxyl, alkyl, aminoalkyl, alkoxy, carboxylic acid, carboxyester, carboxamide, carbamate, sulfonic acid, sulfone, sulfonamide, trifluoromethyl, aryl and heteroaryl, wherein said 5- to 8- membered heterocyclic groups contain one or more heteroatom(s) selected from N, O, and S, with point of attachment being carbon or nitrogen;
Y¹ is absent, or independently selected at each occurrence from the group consisting of-C(O)-, -C(O)NH-, -S(O)₂-, -S(O)₂NH-,-C(O)O-, -C(NH)NH-;
wherein said halogen is selected from the group consisting of fluorine, chlorine and bromine;
or an enantiomer thereof or a mixture of its enantiomers or its pharmaceutically acceptable salt.

2. A compound according to claim 1, wherein n is 2 and at least one occurrence of Z⁰, Z¹ and Z² is selected from N, NR⁴, O and S such that a 6-membered heterocycle is formed.

3. A compound according to claim 1 or 2, wherein the pharmaceutically acceptable salt is selected from the group consisting of the hydrochloride, hydrobromide, hydroiodide, nitrate, sulfate, bisulfate, phosphate, acid phosphate, isonicotinate, acetate, lactate, salicylate, citrate, tartrate, pantothenate, bitartrate, ascorbate, succinate, maleate, gentisinate, fumarate, gluconate, glucuronate, saccharate, formate, benzoate, glutamate, methanesulfonate, ethanesulfonate, benzensulfonate, p-toluenesulfonate and pamoate.

4. A pharmaceutical composition comprising a therapeutically effective amount of at least one compound according to claim 1.

5. A pharmaceutical composition according to claim 4, wherein said therapeutically effective amount is 0.01 to 1000 mg/kg per day.

6. A pharmaceutical composition according to claim 4 or 5, wherein said composition additionally comprises at least one therapeutic agent selected from the group comprising a chemotherapeutic or anti-proliferative agent, an immunomodulatory or immunosuppressive agent, or an anti-inflammatory agent; or wherein said composition comprises said at least one compound according to formula (A) as the only pharmaceutically active agent.

7. A pharmaceutical composition according to any of claims 4 to 6, wherein said composition is administered parenterally, vaginally, rectally, transdermally, orally or through the otolaryngologal sphere.

8. A pharmaceutical composition according to any of claims 4 to 7, wherein said composition additionally comprises at least one pharmaceutically acceptable excipient selected from the group consisting of flavoring agents, sweetener, lubricants, solubilizers, suspending agents, fillers, glidants, compression aides, binders, tablet-disintegrating agents, effervescing agent, wetting agent encapsulating materials, dyestuff, and mixtures thereof, wherein said carrier is preferably chosen from solid or liquid carriers and optionally sterile.

9. A pharmaceutical composition according to any of claims 4 to 8, wherein said composition is a sterile solution or suspension suitable for parenteral administration by intramuscular, intraperitoneal, intravenous, intrathecal, or subcutaneous injection or perfusion; or wherein said composition is an oral dosage form, preferably a liquid or solid dosage form selected from the group comprising pills, tablets, capsules, sachets, dragees, powders, granules, lozenges, powders for reconstitution, orally disintegrating tablet, films, osmotic controlled release capsule, elixir, emulsion, syrup, suspension, tincture and solutions or powder for inhalation and nebulization, or sublingual administration; or wherein said composition is a transdermal, rectal or vaginal dosage form selected from the group comprising ointments, creams, lotions, liniments, gels, paste, films, suppositories, enemas and pessaries; or wherein said composition is a dosage form for administration through the eyes, ears and nose.

10. A pharmaceutical composition according to any of claim 4 to 9, wherein said composition is an immediate, extended or slow- release formulation.

11. A pharmaceutical composition according to any of claims 4 to 10 for use in the treatment or prevention of a disease selected from the group consisting of myeloid leukemia both acute and chronic, acute lymphoblastic leukemia, chronic lymphocytic leukemia, hairy cell leukemia, a myeloproliferative disease, multiple myeloma, myelodysplastic syndrome, Hodgkin's disease, non-Hodgkin's lymphoma, adenocarcinoma, lymphoma, leukemia of the kidney, Wilm's tumor, renal cell carcinoma, renal pelvis carcinoma, nephroma, teratoma, sarcoma of the kidney, squamous cell carcinoma, transitional cell carcinoma, adenocarcinoma of bladder and urethra, sarcoma of the prostate, seminoma, embryonal carcinoma, teratocarcinoma, choriocarcinoma, sarcoma, interstitial cell carcinoma, fibroma, fibroadenoma, adenomatoid tumors, lipoma of the testis, angiosarcoma, fibrosarcoma, rhabdomyosarcoma, liposarcoma, myxoma, rhabdomyoma, lipoma and teratoma of the heart, astrocytoma, medulloblastoma, glioma, ependymoma, germinoma, glioblastoma multiform, oligodendroglioma, schwannoma, retinoblastoma, congenital tumors of the brain, neurofibroma, meningioma, sarcoma of the spinal cord, osteoma, hemangioma, granuloma, xanthoma, osteitis deformians of the skull, meningiosarcoma, gliomatosis of the meninges, alveolar carcinoma, bronchial adenoma, chondromatous hanlartoma, mesothelioma of the bronchus, carcinoid tumors, Karposi's sarcoma, leiomyoma, lipoma, fibroma of the small bowel, tubular adenoma, villous adenoma, hamartoma, leiomyoma of the large bowel, leiomyosarcoma, lymphoma of the esophagus, carcinoma, leiomyosarcoma of the stomach, ductal adenocarcinoma, insulinoma, glucagonoma, gastrinoma, vipoma of the pancreas, hepatocellular carcinoma, cholangiocarcinoma, hepatoblastoma, hepatocellular adenoma, hemangioma of the liver, osteogenic sarcoma, malignant fibrous histiocytoma, chondrosarcoma, Ewing's sarcoma, malignant lymphoma including reticulum cell sarcoma, malignant giant cell tumor chondroma, osteochondroma including osteocartilaginous exostoses, benign chondroma, chondroblastoma, chondromyxofibroma, osteoid osteoma, giant cell tumors, endometrial carcinoma, cervical carcinoma, pre-tumor cervical dysplasia, ovarian carcinoma including serous cystadenocarcinoma, mucinous cystadenocarcinoma, granulosa-thecal cell tumors, Sertol/Leydig cell tumors, dysgerminoma, malignant teratoma of the ovary, intraepithelial carcinoma, melanoma of the vulva, clear cell carcinoma, botryoid sarcoma including embryonal rhabdomyosarcoma of the vagina, fallopian tubes carcinoma, malignant melanoma, basal cell carcinoma, moles dysplastic nevi, angioma, dermatofibroma, keloids, bone marrow transplant rejection, rheumatoid arthritis, psoriasis, type I diabetes mellitus and multiple sclerosis.

12. Use of a compound according to claim 1 for the preparation of a pharmaceutical composition according to any of claims 4 to 11.

13. A method for modulating or regulating serine/threonine or tyrosine kinases, preferably selected from the group of PIM, HIPK, DYRK, CLK, CDK, FLT, PKG, Haspin, MER, TAO, MNK, TRK kinases, wherein said serine/threonine or tyrosine kinases are exposed to an effective amount of at least one compound according to claim 1, wherein said method is performed outside the human or animal body.

14. A process for the preparation of a compound according to claim 1, wherein said process comprises: reacting of an unsubstituted or substituted 2,4,5,6,7-pentahalogenobenzimidazole with a suitable amine at elevated temperature, wherein the reactive substituents are optionally protected with suitable protecting groups and wherein the resulting product is preferably subjected to purification by crystallization or chromatography according to the reaction, wherein said halogen is selected from the group consisting of fluorine, chlorine and bromine.

## Patentansprüche

1. Eine Verbindung der Formel (A): 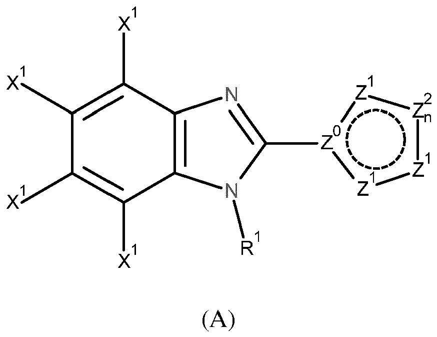 wobei:
X¹ jeweils unabhängig bei jedem Auftreten ausgewählt ist aus F, Cl, oder Br;
Z⁰ ausgewählt ist aus C, CH und N;
Z¹ jeweils unabhängig bei jedem Auftreten ausgewählt ist aus CR², CHR³, N, NR⁴ und O;
Z² jeweils unabhängig bei jedem Auftreten ausgewählt ist aus CR², CHR³, N, NR⁴, O und S;
n 1, 2, 3 oder 4 ist, um einen 5-, 6-, 7- oder 8-gliedrigen Kohlenstoffzyklus oder Heterozyklus zu bilden, welcher gesättigt oder ungesättigt, aber nicht aromatisch ist;
Z¹ und Z² optional unabhängig zusammengenommen sind, um einen zweiten und optional einen dritten fusionierten Ring zu bilden, um mindestens einen 4-, 5-, 6- oder 7-gliedrigen Kohlenstoffzyklus oder Heterozyklus zu formen, welcher gesättigt oder ungesättigt oder aromatisch ist und welcher optional substituiert ist mit einem oder mehreren Substituenten ausgewählt aus H, Halogen, Amino, Hydroxy, Aminoalkyl, Alkoxy, Carbonsäure, Carbonsäureester, Carboxamid, Carbamat, Sulfonsäure, Sulfonamid und Trifluormethyl, wobei besagte 4- bis 7-gliedrige heterozyklische Gruppen ein oder mehrere Heteroatom(e) ausgewählt aus N, O und S enthalten;
R¹ ausgewählt ist aus der Gruppe bestehend aus H, Methyl, Carbonsäureester, Carboxamid, Sulfonamid, -(C₂₋₆Alkyl)R_{A} und 5- bis 8-gliedrigen kohlenstoffzyklischen und heterozyklischen Gruppen, welche gesättigt, ungesättigt oder aromatisch sind, welche optional substituiert sind mit einem oder mehreren Substituenten ausgewählt aus H, Halogen, Amino, Hydroxy, Aminoalkyl, Alkoxy, Carbonsäure, Carbonsäureester, Carboxamid, Carbamat, Sulfonsäure, Sulfonamid und Trifluormethyl, wobei besagte 5- bis 8-gliedrige heterozyklische Gruppen ein oder mehrere Heteroatom(e) ausgewählt aus N, O und S enthalten, wobei der Anknüpfungspunkt Kohlenstoff ist, wobei besagtes -(C₂₋₆Alkyl)R_{A} optional verzweigt und weiter substituiert ist mit einem oder mehreren Substituenten ausgewählt aus Oxo, Hydroxyl und Amino;
R² jeweils unabhängig ausgewählt ist aus der Gruppe bestehend aus H, Halogen, Amino, Hydroxy, Oxo, Aminoalkyl, Alkoxy, Carbonyl, Carbonsäure, Carbonsäureester, Carboxamid, Carbamat, Sulfonsäure, Sulfonamid, -Y¹(C₁₋₆Alkyl)R_{A} und Y¹R_{B}, wobei besagtes - Y¹(C₁₋₆Alkyl)R_{A} optional verzweigt und weiter substituiert ist mit einem oder mehreren Substituenten ausgewählt aus Oxo, Hydroxyl und Amino;
R³ jeweils unabhängig ausgewählt ist aus der Gruppe bestehend aus H, Amino, Hydroxy, Aminoalkyl, Alkoxy, Carbonsäure, Carbonsäureester, Carboxamid, Carbamat, Sulfonsäure, Sulfonamid, Tufluormethyl, -Y¹(C₁₋₆Alkyl)R_{A} und Y¹R_{B}, wobei besagtes -Y¹(C₁₋₆Alkyl)R_{A} optional verzweigt und weiter substituiert ist mit einem oder mehreren Substituenten ausgewählt aus Oxo, Hydroxyl und Amino;
R⁴ jeweils unabhängig ausgewählt ist aus der Gruppe bestehend aus H, Carbonsäureester, Carboxamid, Carbamat, Sulfonamid, Amidin, -Y¹(C₁₋₆Alkyl)R_{A} und R_{B} mit der Maßgabe, dass der Anknüpfungspunkt an R_{B} Kohlenstoff ist, wobei besagtes -Y¹(C₁₋₆Alkyl)R_{A} optional verzweigt und weiter substituiert ist mit einem oder mehreren Substituenten ausgewählt aus Oxo, Hydroxy, Nitril und Amino;
R_{A} jeweils unabhängig ausgewählt ist aus der Gruppe bestehend aus H, Amino, Hydroxy, Aminoalkyl, Alkoxy, Carbonsäure, Carbonsäureester, Carboxamid, Carbamat, Sulfonsäure, Sulfonamid, Trifluormethyl, 5- bis 9-gliedrigen mono- oder bizyklischen kohlenstoffzyklischen und heterozyklischen Gruppen, welche gesättigt, ungesättigt oder aromatisch sind, welche optional substituiert sind mit einem oder mehreren Substituenten ausgewählt aus H, Halogen, Amino, Hydroxy, Alkyl, Aminoalkyl, Alkoxy, Carbonsäure, Carbonsäureester, Carboxamid, Carbamat, Sulfonsäure, Sulfonamid, Trifluormethyl, substituiertes oder unsubstituiertes Aryl und Heteroaryl, wobei besagte 5- bis 9-gliedrige heterozyklische Gruppen ein oder mehrere Heteroatom(e) ausgewählt aus N, O und S, mit Kohlenstoff oder Stickstoff als Anknüpfungspunkt, enthalten;
R_{B} jeweils unabhängig ausgewählt ist aus der Gruppe bestehend aus 5- bis 8-gliedrigen kohlenstoffzyklischen und heterozyklischen Gruppen, welche gesättigt, ungesättigt oder aromatisch sind, welche optional substituiert sind mit einem oder mehreren Substiuenten ausgewählt aus H, Halogen, Amino, Hydroxy, Alkyl, Aminoalkyl, Alkoxy, Carbonsäure, Carbonsäureester, Carboxamid, Carbamat, Sulfonsäure, Sulfon, Sulfonamid, Trifluormethyl, Aryl und Heteroaryl, wobei besagte 5- bis 8-gliedrige heterozyklische Gruppen ein oder mehrere Heteroatom(e) ausgewählt aus N, O und S, mit Kohlenstoff oder Stickstoff als Anknüpfungspunkt, enthalten;
Y¹ abwesend ist oder jeweils unabhängig ausgewählt ist aus der Gruppe bestehend aus -C(O)-, -C(O)NH-, -S(O)₂-, -S(O)₂NH-, -C(O)O-, -C(NH)NH-; wobei besagtes Halogen ausgewählt ist aus der Gruppe bestehend aus Fluor, Chlor und Brom;
oder ein Enantiomer davon oder eine Mischung ihrer Enantiomere
oder ihr pharmazeutisch akzeptables Salz.

2. Eine Verbindung gemäß Anspruch 1, wobei n 2 ist und mindestens ein Auftreten von Z⁰, Z¹ und Z² ausgewählt ist aus N, NR⁴, O und S, sodass ein 6-gliedriger Heterozyklus gebildet wird.

3. Eine Verbindung gemäß Anspruch 1 oder 2, wobei das pharmazeutisch akzeptable Salz ausgewählt ist aus der Gruppe bestehend aus dem Hydrochlorid, Hydrobromid, Hydroiodid, Nitrat, Sulfat, Bisulfat, Phosphat, Säurephosphat, Isonicotinat, Acetat, Laktat, Salicylat, Citrat, Tartrat, Pantothenat, Bitartrat, Ascorbat, Succinat, Maleat, Gentisinat, Fumarat, Gluconat, Glucuronat, Saccharat, Format, Benzoat, Glutamat, Methansulfonat, Ethansulfonat, Benzolsulfonat, p-Toluolsulfonat und Pamoat.

4. Eine pharmazeutische Zusammensetzung umfassend eine therapeutisch wirksame Menge mindestens einer Verbindung gemäß Anspruch 1.

5. Eine pharmazeutische Zusammensetzung gemäß Anspruch 4, wobei besagte therapeutisch wirksame Menge 0,01 bis 1000 mg/kg pro Tag ist.

6. Eine pharmazeutische Zusammensetzung gemäß Anspruch 4 oder 5, wobei besagte Zusammensetzung zusätzlich mindestens einen therapeutischen Wirkstoff umfasst, ausgewählt aus der Gruppe umfassend einen chemotherapeutischen oder anti-proliferativen Wirkstoff, einen immunmodulatorischen oder immunsuppressiven Wirkstoff, oder einen antiinflammatorischen Wirkstoff; oder wobei besagte Zusammensetzung besagte mindestens eine Verbindung gemäß Formel (A) als den einzigen pharmazeutischen Wirkstoff umfasst.

7. Eine pharmazeutische Zusammensetzung gemäß einem der Ansprüche 4 bis 6, wobei besagte Zusammensetzung parenteral, vaginal, rektal, transdermal, oral oder durch den otolaryngologalen Bereich verabreicht wird.

8. Eine pharmazeutische Zusammensetzung gemäß einem der Ansprüche 4 bis 7, wobei besagte Zusammensetzung zusätzlich mindestens einen pharmazeutisch akzeptablen Hilfsstoff umfasst, ausgewählt aus der Gruppe bestehend aus Geschmacksstoffen, Süßstoffen, Schmierstoffen, Lösungsstoffen, Suspensionssmitteln, Füllstoffen, Fließmitteln, Kompressionshilfsstoffen, Bindern, Tabletten-auflösenden Stoffen, Brausestoffen, Benetzungsmittel-einschließenden Materialien, Farbstoffen und Mischungen davon, wobei besagter Träger vorzugsweise aus festen oder flüssigen Trägern und optional sterilen ausgewählt ist.

9. Eine pharmazeutische Zusammensetzung gemäß einem der Ansprüche 4 bis 8, wobei besagte Zusammensetzung eine sterile Lösung oder Suspension ist geeignet zur parenteralen Verabreichung durch intramuskuläre, intraperitoneale, intravenöse, intrathekale oder subkutane Injektion oder Perfusion; oder wobei besagte Zusammensetzung eine orale Darreichungsform ist, vorzugsweise eine flüssige oder feste Darreichungsform ausgewählt aus der Gruppe umfassend Pillen, Tabletten, Kapseln, Dosierungsbeutel, Dragees, Pulver, Granulaten, Lutschtabletten, Pulver zur Rekonstitution, sich im Mund auflösende Tablette, Filme, osmotische Retardkapsel, Elixier, Emulsion, Sirup, Suspension, Tinktur und Lösungen oder Pulver zur Inhalation und Zerstäubung, oder sublinguale Verabreichung; oder wobei besagte Zusammensetzung eine transdermale, rektale oder vaginale Darreichungsform ist ausgewählt aus der Gruppe umfassend Salben, Cremes, Lotionen, Einreibemitteln, Gelen, Pasten, Filmen, Zäpfchen, Einläufen und Vaginalzäpfchen; oder wobei besagte Zusammensetzung eine Darreichungsform ist zur Verabreichung durch die Augen, Ohren und Nase.

10. Eine pharmazeutische Zusammensetzung gemäß einem der Ansprüche 4 bis 9, wobei besagte Zusammensetzung eine unmittelbar, verzögernd oder langsam freisetzende Formulierung ist.

11. Pharmazeutische Zusammensetzung gemäß einem der Ansprüche 4 bis 10 zur Behandlung oder Prävention einer Krankheit ausgewählt aus der Gruppe bestehend aus myeloische Leukämie sowohl akut als auch chronisch, akuter lymphoblastische Leukämie, chronische lymphozytische Leukämie, Haarzellenleukämie, einer myeloproliferativen Krankheit, multiples Myelom, myelodysplastisches Syndrom, Morbus Hodgkin, non-Hodgkin-Lymphom, Adenokarzinom, Lymphom, Leukämie der Niere, Wilm's Tumor, Nierenzellkarzinom, Nierenbeckenkarzinom, Nephrom, Teratom, Nierensarkom, Plattenepithelkarzinom, Übergangszellkarzinom, Adenokarzinom der Blase und Harnröhre, Prostatasarkom, Seminom, embryonales Karzinom, Teratokarzinom, Choriokarzinom, Sarkom, interstitielles Zellkarzinom, Fibrom, Fibroadenom, adenomatoide Tumore, Hoden-Lipom, Angiosarkom, Fibrosarkom, Rhabdomyosarkom, Liposarkom, Myxom, Rhabdomyom, Lipom und Teratom des Herzens, Astrozytom, Medulloblastom, Gliom, Ependymom, Germinom, Glioblastoma multiforme, Oligodendrogliom, Schwannom, Retinoblastom, angeborene Hirntumore, Neurofibrom, Meningiom, Rückenmarkssarkom, Osteom, Hämangiom, Granulom, Xanthom, Osteitisdeformanz des Schädels, Meningiosarkom, Gliomatose der Hirnhäute, alveoläres Karzinom, Bronchialadenom, chondromatöses Hanlartom, Mesotheliom der Bronchien, karzinoide Tumore, Karposi-Sarkom, Leiomyom, Lipom, Fibrom des Dünndarms, tubuläres Adenom, villöses Adenom, Hamartom, Leiomyom des Dickdarms, Leiomyosarkom, Speiseröhrenlymphom, Karzinom, Leiomyosarkom des Magens, duktales Adenokarzinom, Insulinom, Glucagonom, Gastrinom, Bauchspeicheldrüsenvipom, Leberzellenkarzinom, Cholangiokarzinom, Hepatoblastom, Leberzellenadenom, Leberhemangiom, osteogenes Sarkom, bösartiges fibröses Histiocytom, Chondrosarkom, Ewing-Sarkom, bösartiges Lymphom einschließlich Retikulumzellsarkom, bösartiges Riesenzelltumorchondrom, Osteochondrom einschließlich osteocartilaginären Exostose, gutartiges Chondrom, Chondroblastom, Chondromyxofibrom, Osteoidosteom, Riesenzelltumore, Endometriumkarzinom, Zervixkarzinom, vor-tumorartige Gebärmutterhalsdysplasie, Eierstockkarzinom einschließlich seröses Zystadenokarzinom, mucinöses Zystadenokarzinom, Granulosa-Theka-Zelltumore, Sertol/Leydig-Zelltumore, Dysgerminom, bösartiges Eierstockteratom, intraepitheliales Karzinom, Melanom der Vulva, klarzelligem Karzinom, traubenförmiges Sarkom einschließlich embryonales Rhabdomyosarkom der Vagina, Eileiterkarzinom, bösartiges Melanom, Basalzellenkarzinom, dysplastische Leberflecken, Angiom, Dermatofibrom, Keloiden, Knochenmarkstransplantationsabstoßung, rheumatoider Arthritis, Schuppenflechte, Typ I Diabetes mellitus und multiple Sklerose.

12. Verwendung einer Verbindung gemäß Anspruch 1 zur Herstellung einer pharmazeutischen Zusammensetzung gemäß einem der Ansprüche 4 bis 11.

13. Ein Verfahren zum Modulieren oder Regulieren von Serin/Threonin- oder Tyrosinkinasen, vorzugsweise ausgewählt aus der Gruppe aus PIM, HIPK, DYRK, CLK, CDK, FLT, PKG, Haspin, MER, TAO, MNK, TRK Kinasen, wobei besagte Serin/Threonin- oder Tyrosinkinasen einer wirksamen Menge mindestens einer Verbindung gemäß Anspruch 1 ausgesetzt sind, wobei besagtes Verfahren außerhalb des menschlichen oder tierischen Körpers durchgeführt wird.

14. Ein Verfahren zur Herstellung einer Verbindung gemäß Anspruch 1, wobei besagtes Verfahren umfasst: Reagieren eines unsubstituierten oder substituierten 2,4,5,6,7-Pentahalogenbenzimidazols mit einem geeigneten Amin bei erhöhter Temperatur, wobei die reaktiven Substituenten optional mit geeigneten Schutzgruppen geschützt sind und wobei das entstehende Produkt vorzugsweise einer Reinigung durch Kristallisation oder Chromatographie gemäß der Reaktion unterzogen wird, wobei besagtes Halogen ausgewählt ist aus der Gruppe bestehend aus Fluor, Chlor und Brom.

## Revendications

1. Composé de formule (A) : 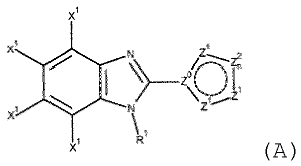 dans laquelle :
X¹ est choisi indépendamment à chaque occurrence parmi les atomes F, Cl, ou Br. ;
Z⁰ est choisi parmi un atome C, un groupe CH, et un atome N ;
Z¹ est choisi indépendamment à chaque occurrence parmi les groupes CR², CHR³, N, NR⁴ et O ;
Z² est choisi indépendamment à chaque occurrence parmi les groupes CR², CHR³, N, NR⁴, 0, et S ;
n est égal à 1, 2, 3, ou 4 pour former un cycle carboné ou un hétérocycle annulaire à 5, 6, 7, ou 8 chaînons, qui est saturé ou insaturé, mais non aromatique ;
Z¹ et Z² sont de manière optionnelle indépendamment assemblés pour former un second, et, de manière optionnelle, un troisième cycle, fusionné pour former au moins un cycle carboné ou un hétérocycle annulaire à 4, 5, 6, ou 7 chaînons, qui est saturé ou insaturé, ou aromatique, et qui est de manière optionnelle substitué par un ou plusieurs substituant(s) choisis parmi l'atome H, les atomes des halogènes, les groupements amine, hydroxyle, aminoalkyles, alcoxy, les acides carboxyliques, les carboxyesters, les carboxamides, les carbamates, les acides sulfoniques, les sulfonamides et les trifluorométhyles, où lesdits groupes hétérocycliques de 4 à 7 chaînons contiennent un ou plusieurs hétéroatome(s) choisis parmi N, 0, et S ;
R¹ est choisi dans le groupe formé par l'atome H, les groupements méthyle, les carboxyesters, carboxamides, sulfonamides, les groupes -(alkyle en C₂₋₆)R_{A}, et les groupes carbocycliques et hétérocycliques de 5 à 8 chaînons, qui sont saturés, insaturés, ou aromatiques, qui sont de manière optionnelle substitués par un ou plusieurs substituant(s) choisis parmi l'atome H, les atomes d'halogènes, les groupes amine, hydroxyle, aminoalkyles, alcoxy, les acides carboxyliques, les carboxylesters, les carboxamides, les carbamates, les acides sulfoniques, les sulfonamides et les trifluorométhyles,
où lesdits groupes hétérocycliques de 5 à 8 chaînons contiennent un ou plusieurs hétéroatome(s) choisis parmi N, 0, et S, qui est lié (sont liés) au carbone, dans lequel ledit groupe - (alkyle en C₂₋₆)R_{A} est ramifié de manière optionnelle et en outre substitué par un ou plusieurs substituant(s) choisi(s) parmi les groupements oxo, hydroxyle, et amine ;
R² est choisi indépendamment à chaque occurrence dans le groupe constitué par l'atome H, les atomes d'halogènes, les groupes amine, hydroxyle, oxo, aminoalkyle, alcoxy, carbonyle, les acides carboxyliques, les carboxyesters, les carboxamides, les carbamates, les acides sulfoniques, les sulfonamides, les groupes -Y¹(alkyle en C₁₋₆)R_{A}, et
Y¹ R_{B}, où ledit groupe -Y¹(alkyle en C₁₋₆)R_{A} est ramifié de manière optionnelle et en outre substitué par un ou plusieurs substituant(s) choisi(s) parmi les groupements oxo, hydroxyle, et amine ;
R³ est choisi indépendamment à chaque occurrence dans le groupe constitué par l'atome H, les groupes amine, hydroxyle, aminoalkyle, alcoxy, les acides carboxyliques, les carboxyesters, les carboxamides, les carbamates, les acides sulfoniques, les sulfonamides, les trifluorométhyles, les groupes -Y¹(alkyle en C₁₋₆)R_{A}, et Y¹ R_{B}, où ledit groupe -Y¹ (alkyle en C₁₋₆)R_{A} est ramifié de manière optionnelle et en outre substitué par un ou plusieurs substituant(s) choisi(s) parmi les groupements oxo, hydroxyle, et amine ;
R⁴ est choisi indépendamment à chaque occurrence dans le groupe constitué par l'atome H, les groupes carboxyesters, carboxamides, carbamates, sulfonamides, amidines, -Y¹(alkyle en C₁₋₆)R_{A}, et R_{B}, à la condition que R_{B} soit lié au carbone, dans lequel le groupe -Y¹ (alkyle en C₁₋₆)R_{A} est ramifié de manière optionnelle et en outre substitué par un ou plusieurs substituant(s) choisi(s) parmi les groupements oxo, hydroxyle, nitrile et amine ;
R_{A} est choisi indépendamment à chaque occurrence dans le groupe constitué par l'atome H, les groupes amine, hydroxyle, aminoalkyle, alcoxy, acides carboxyliques, carboxyesters, carboxyamides, les carbamates, les acides sulfoniques, les sulfonamides, les trifluorométhyles, les groupes carbocycliques et hétérocycliques en forme de monocycle ou de bicycle comprenant de 5 à 9 chaînons, qui sont saturés, insaturés, ou aromatiques, qui sont substitués de manière optionnelle par un ou plusieurs substituant(s) choisi(s) parmi l'atome H, les atomes d'halogènes, les groupes amine, hydroxyle, alkyle, aminoalkyle, alcoxy, les acides carboxyliques, les carboxyesters, les carboxyamides, les carbamates, les acides sulfoniques, les sulfonamides, les trifluorométhyles, les aryles et les hétéroaryles substitués ou non substitués, où lesdits groupes hétérocycliques formés de 5 à 9 chaînons contiennent un ou plusieurs hétéroatome(s) choisis parmi N, O, et S, qui est lié (sont liés) aux atomes de carbone ou d'azote ;
R_{B} est choisi indépendamment à chaque occurrence dans l'ensemble constitué par les groupes carbocycliques et hétérocycliques formés de 5 à 8 chaînons, qui sont saturés, insaturés, ou aromatiques, qui sont substitués de manière optionnelle par un ou plusieurs substituant(s) choisi(s) parmi l'atome H, les atomes d'halogènes, les groupes amine, hydroxyle, alkyle, aminoalkyle, alcoxy, les acides carboxyliques, les esters carboxyliques, les amides carboxyliques, les carbamates, les acides sulfoniques, les sulfones, les sulfonamides, les trifluorométhyles, les aryles et les hétéroaryles, où lesdits les groupes hétérocycliques formés de 5 à 8 chaînons contiennent un ou plusieurs hétéroatome(s) choisis parmi N, O, et S, qui est lié (sont liés) au carbone ou à l'azote ;
Y¹ est manquant, ou choisi indépendamment à chaque occurrence dans le groupe constitué par les groupements -C(O)-, -C(O)NH-, -S(O)₂-, -S(O)₂NH-, -C(O)O-, -C(NH)NH- ; où ledit halogène est choisi dans le groupe formé par le fluor, le chlore et le brome ; ou un énantiomère de ceux-ci ou un mélange de ces énantiomères ou de leurs sels pharmaceutiquement acceptables.

2. Composé selon la revendication 1, dans lequel n prend la valeur 2 et au moins une occurrence de Z⁰, Z¹ et Z² est choisie parmi N, NR⁴, 0 et S de telle sorte que cela forme un hétérocycle à 6 chaînons.

3. Composé selon la revendication 1 ou 2, dans lequel le sel pharmaceutiquement acceptable est choisi dans le groupe constitué par les groupes hydrochlorure, hydrobromure, hydroiodure, nitrate, sulfate, bisulfate, phosphate, phosphate d'acide, isonicotinate, acétate, lactate, salicylate, citrate, tartrate, pantothénate, bitartrate, ascorbate, succinate, maléate, gentisinate, fumarate, gluconate, glucuronate, saccharate, formate, benzoate, glutamate, méthanesulfonate, éthanesulfonate, benzènesulfonate, p-toluènesulfonate et pamoate.

4. Composition pharmaceutique comprenant une quantité thérapeutiquement efficace d'au moins un composé selon la revendication 1.

5. Composition pharmaceutique selon la revendication 4, dans laquelle ladite quantité thérapeutiquement efficace se situe entre 0,01 et 1000 mg/kg par jour.

6. Composition pharmaceutique selon la revendication 4 ou 5, dans laquelle ladite composition comprend à titre complémentaire au moins un agent thérapeutique choisi dans le groupe comprenant un agent chimio-thérapeutique ou antiprolifératif, un agent immuno-modulateur ou immunosuppresseur, ou un agent anti-inflammatoire ; ou dans laquelle ladite composition comprend au moins un composé selon la formule (A) en tant que seul agent pharmaceutiquement actif.

7. Composition pharmaceutique selon l'une quelconque des revendications 4 à 6, dans laquelle ladite composition est administrée par voie parentérale, vaginale, rectale, transdermique, orale ou par la sphère oto-rhino-laryngée.

8. Composition pharmaceutique selon l'une quelconque des revendications 4 à 7, où ladite composition comprend à titre complémentaire au moins un excipient pharmaceutiquement acceptable choisi dans le groupe constitué par les agents aromatisants, les édulcorants, les lubrifiants, les agents de solubilisation, les agents de mise en suspension, les charges, les agents de glissement, les auxiliaires de compression, les liants, les agents de désagrégation des comprimés, les agents effervescents, les matériaux d'encapsulation d'agent mouillant, les colorants, et les mélanges de ceux-ci, où le vecteur est de préférence choisi à partir de vecteurs solides ou liquides et, de manière optionnelle, stériles.

9. Composition pharmaceutique selon l'une quelconque des revendications 4 à 8, où ladite composition est une solution stérile ou une suspension indiquée pour une administration parentérale par injection intramusculaire, intrapéritonéale, intraveineuse, intrathécale, ou sous-cutanée ou par perfusion ; ou ladite composition est une forme posologique orale, de préférence une forme posologique liquide ou solide choisie dans le groupe comprenant les pilules, les comprimés, les capsules, les sachets, les dragées, les poudres, les granulés, les pastilles, les poudres pour reconstitution, les comprimés à désintégration orale, les films, les capsules osmotiques à libération contrôlée, les élixirs, les émulsions, les sirops, les suspensions, les teintures et les solutions ou les poudres pour les inhalations et les nébulisations, ou l'administration sublinguale ; ou ladite composition est une forme posologique transdermique, rectale ou vaginale choisie dans le groupe comprenant les pommades, les crèmes, les lotions, les liniments, les gels, les pâtes, les suppositoires, les lavements et les pessaires ; ou ladite composition est une forme posologique pour une administration à travers les yeux, les oreilles et le nez.

10. Composition pharmaceutique selon l'une quelconque des revendications 4 à 9, dans laquelle ladite composition est une formulation à libération immédiate, prolongée ou lente.

11. Composition pharmaceutique selon l'une quelconque des revendications 4 à 10 destinée au traitement ou à la prévention d'une maladie choisie dans le groupe constitué par la leucémie myéloïde à la fois aigüe et chronique, la leucémie lymphoblastique aigüe, la leucémie lymphocytique chronique, la leucémie à tricholeucocytes, la maladie myéloproliférative, le myélome multiple, le syndrome myélodysplasique, la maladie de Hodgkin, le lymphome non hodgkinien, l'adénocarcinome, le lymphome, la leucémie du rein, la tumeur de Wilm, le carcinome de la cellule rénale, le carcinome rénal pelvien, le néphrome, le tératome, le sarcome du rein, le carcinome épidermoïde, le carcinome des cellules transitionnelles, l'adénocarcinome de la vessie et de l'urètre, le sarcome de la prostate, le séminome, le carcinome embryonnaire, le tératocarcinome, le choriocarcinome, le sarcome, le carcinome des cellules interstitielles, le fibrome, le fibroadénome, les tumeurs adénomatoïdes, le lipome du testicule, l'angiosarcome, le fibrosarcome, le rhabdomyosarcome, le liposarcome, le myxome, le rhabdomyome, le lipome et le tératome cardiaque, l'astrocytome, le médulloblastome, le gliome, l'épendymome, le germinome, le glioblastome multiforme, l'oligodendrogliome, le schwannome, le rétinoblastome, les tumeurs congénitales du cerveau, le neurofibrome, le méningiome, le sarcome de la moelle épinière, l'ostéome, l'hémangiome, le granulome, le xanthome, l'ostéite déformante du crâne, le méningiosarcome, le gliomatose des méninges, le carcinome alvéolaire, l'adénome bronchique, l'hamartome chondromateux, le mésothéliome de la bronche, les tumeurs carcinoïdes, le sarcome de Karposi, le léiomyome, le lipome, le fibrome de l'intestin grêle, l'adénome tubulaire, l'adénome villeux, l'hamartome, le léiomyome du gros intestin, le léiomyosarcome, le lymphome de l'oesophage, le carcinome, le léiomyosarcome de l'estomac, l'adénocarcinome ductal, l'insulinome, le glucagonome, le gastrinome, le vipome du pancréas, le carcinome hépatocellulaire, le cholangiocarcinome, l'hépatoblastome, l'adénome hépatocellulaire, l'hémangiome du foie, le sarcome ostéogénique, l'histiocytome fibreux malin, le chondrosarcome, le sarcome d'Ewing, le lymphome malin y compris le réticulosarcome, le chondrome malin à cellule tumorale géante, l'ostéochondrome y compris les exostoses ostéocartilagineuses, le chondrome bénin, le chondroblastome, le chondromyxofibrome, l'ostéome ostéoide, les tumeurs à cellule géante, le carcinome de l'endomètre, le carcinome cervical, la dysplasie cervicale prétumorale, le carcinome ovarien y compris le cystadénocarcinome séreux, le cystadénome mucineux, les tumeurs à cellules thécales - à cellules de la granulosa, les tumeurs à cellules de Sertoli-Leydig, le dysgerminome, le tératome malin de l'ovaire, le carcinome intraépithélial, le mélanome de la vulve, le carcinome à cellules claires, le sarcome botryoïde y compris le rhabdomyosarcome embryonnaire du vagin, le carcinome des trompes de Fallope, le mélanome malin, le carcinome basocellulaire, les naevi dysplasiques, l'angiome, le dermatofibrome, les chéloïdes, le rejet de la greffe de moelle osseuse, l'arthrite rhumatoïde, le psoriasis, le diabète sucré de type I et la sclérose en plaques.

12. Utilisation d'un composé selon la revendication 1 pour la préparation d'une composition pharmaceutique selon l'une quelconque des revendications 4 à 11.

13. Procédé de modulation ou de régulation des sérine/thréosine ou tyrosine kinases, choisies de préférence dans le groupe des PIM, HIPK, DYRK, CLK, CDK, FLT, PKG, Haspin, MER, TAO, MNK, TRK kinases, dans lequel lesdites sérine/thréosine ou tyrosine kinases sont exposées à une quantité efficace d'au moins un composé selon la revendication 1, dans lequel ledit procédé est exécuté à l'extérieur du corps humain ou du corps de l'animal.

14. Procédé de préparation d'un composé selon la revendication 1, dans lequel ledit procédé comprend: la réaction d'un penta halogéno benzimidazole 2,4,5,6,7 non substitué ou substitué avec une amine appropriée à température élevée, dans lequel les substituants réactifs sont protégés de manière optionnelle par les groupes protecteurs appropriés et dans lequel le produit résultant est de préférence soumis à une purification par cristallisation ou par chromatographie, en fonction de la réaction, dans lequel ledit halogène est choisi dans le groupe constitué par le fluor, le chlore et le brome.
